(19) Europäisches Patentamt
European Patent Office
Office européen des brevets

(11) EP 0 512 415 B1

(12) EUROPÄISCHE PATENTSCHRIFT

(45) Veröffentlichungstag und Bekanntmachung des Hinweises auf die Patenterteilung: **06.08.1997 Patentblatt 1997/32**

(51) Int. Cl.[6]: **C07C 231/18**, C07D 241/04, C07D 211/44

(21) Anmeldenummer: **92107395.3**

(22) Anmeldetag: **30.04.1992**

# (54) Verfahren zur enantioselektiven Synthese von 2(R)-Benzylbernsteinsäuremonoamid-Derivaten

Process for the enantioselective synthesis of 2(R)-benzyl succanamide derivatives

Procédé pour la préparation enantiosélective des dérivés de 2(R) benzyl succinamide

(84) Benannte Vertragsstaaten:
**AT BE CH DE DK ES FR GB GR IT LI LU NL PT SE**

(30) Priorität: **03.05.1991 DE 4114401**

(43) Veröffentlichungstag der Anmeldung:
**11.11.1992 Patentblatt 1992/46**

(73) Patentinhaber:
**HOECHST AKTIENGESELLSCHAFT**
**65926 Frankfurt am Main (DE)**

(72) Erfinder:
- **Lerch, Ulrich, Dr.**
  **W-6238 Hofheim am Taunus (DE)**
- **Jendralla, Joachim-Heiner, Dr.**
  **W-6230 Frankfurt am Main 80 (DE)**
- **Seuring, Bernhard, Dr.**
  **W-6238 Hofheim am Taunus (DE)**
- **Henning, Rainer, Dr.**
  **W-6234 Hattersheim am Main (DE)**

(56) Entgegenhaltungen:
- **CHEMICAL ABSTRACTS, Bd. 114, Nr. 1, 7. Januar 1991, Columbus, Ohio, US; Zusammenfassung Nr. 7252q, A. TERAJIMA et al: "Preparation of (R)-2-(1-naphthylmethyl)succinic acid as an intermediate for renin-inhibitory peptides", Seite 716; & JP-A-02 157 243**
- **CHEMICAL ABSTRACTS, Bd. 114, nr. 5, 4. Februar 1991, Columbus, Ohio, US; Zusammenfassung Nr. 43168a, K. ACHINAMI et al: "Preparation of asymmetric trans-4,5-bis[bis(4-methoxy-3,5-dimethylphenyl)phosphinomethyl]-2,2-dimethyl-1,3-dioxolane", Seite 780; JP-A-02 091 090**
- **CHEMICAL ABSTRACTS, Bd. 113, Nr. 7, 13. August 1990, Columbus, Ohio, US; Zusammenfassung Nr. 59204z, K. ACHINAMI et al: "Preparation of optically active 2-(1-naphthylmethyl)succinic acid or its monoamide with piperidine, morpholine or dialkylamine", Seite 708; & JP-A-02 022 244**
- **TETRAHEDRON LETTERS, Bd. 28, Nr. 17, Oxford, GB, Seiten 1905-1908, H. KAWANO et al: "Ruthenium(II)-Binap Complex Catalyzed Asymmetric Hydrogenation of Unsaturated Dicarboxlic Acids"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 512 415 B1

Printed by Rank Xerox (UK) Business Services
2.14.12/3.4

## Beschreibung

Die Verbindungen der allgemeinen Formel (1) sind wertvolle Zwischenprodukte zur Synthese von pharmazeutischen Wirkstoffen, insbesondere von Renin-hemmenden Verbindungen, wie sie z. B. in DE-A 39 30 397 und DE-A 39 33 096 vorgestellt werden. Die in Formel (1) beschriebenen Verbindungen wurden bisher - soweit bekannt - z. B. nach J.J. Plattner et al. [J. Med. Chem. Bd. 31 2277 (1988), Schema V] durch asymmetrische Substitution des Enolats eines homochiralen 3-Phenyl-propionyloxazolidinons an Bromessigsäureester als Schlüsselschritt erhalten.

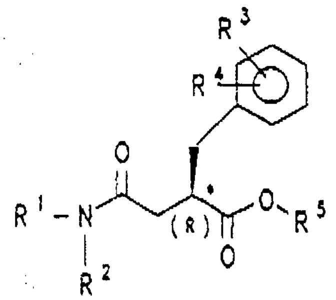

(1)

In Chemical Abstracts, Vol. 114, No. 1, 1991, Abstract No. 7252q wird ein Verfahren zur Herstellung von 2(R)-(1-Naphthylmethyl)bernsteinsäure als Zwischenprodukt für Renininhibitor-Peptide offenbart. In Chemical Abstracts, Vol. 113, No. 7, 1990, Abstract No. 59204z ist die Herstellung von optisch aktiver 2-(1-Naphthylmethyl)bernsteinsäure oder ihrer Monoamide beschrieben, wobei der Rhodium-Komplex des gut zugänglichen (-)-(2S,4S)-BPPM eingesetzt wird, so daß das Hydrierprodukt in der (S)-Konfiguration erhalten wird, was wiederum für Renininhibitoren unbrauchbar ist. Kawano et al. (Tetrahedron Letters, Vol. 28, No. 17. (1987) 1905-1908) geben an, daß man einen Hydrierkatalysator ausreichender Enantioselektivität mit einem Ruthenium(II)-BINAP-Komplex erhält.

Gegenstand der vorliegenden Erfindung ist ein Verfahren zur Herstellung von speziell konfigurierten optisch aktiven Verbindungen der allgemeinen Formel (1), in der

$R^1$ und $R^2$

a) gleich oder verschieden sind und geradkettiges oder verzweigtes Alkyl mit 1 bis 7 Kohlenstoffatomen oder Cycloalkyl mit 5 bis 7 Kohlenstoffatomen bedeuten, wobei die genannten Gruppen substituiert sein können mit bis zu drei gleichen oder verschiedenen Resten aus der Reihe

- $(C_1$-$C_7)$-Alkyl
- Hydroxy (geschützt oder ungeschützt)
- $(C_1$-$C_7)$-Alkoxy
- Amino (geschützt oder ungeschützt)
- $(C_1$-$C_7)$-Alkylamino (geschützt oder ungeschützt)
- Di-$(C_1$-$C_7)$-Alkylamino oder

$R^1$ und $R^2$ gemeinsam können unter Ausbildung eines 5- bis 7-gliedrigen Ringes, von dem 0, 1 oder 2 Ringglieder gleich oder verschieden aus Sauerstoff- oder Stickstoffatomen bestehen, miteinander verbunden sein, der gegebenenfalls mit den obengenannten Resten substituiert sein kann,

b) $R^1$ und $R^2$ können auch die Bedeutung von Phenylgruppen haben, die gegebenenfalls substituiert sein können mit Gruppen, die der unten angegebenen Bedeutung von $R^3$ und $R^4$ entsprechen,

$R^3$ und $R^4$ gleich oder verschieden sind und Wasserstoff, Trifluormethyl, Halogen oder die bei $R^1$ und $R^2$ unter a) genannten Substituenten bedeuten und

$R^5$ ein Wasserstoffatom bedeutet,

wobei man

a) ein Phenylitaconsäure-Derivat der Formel (4)

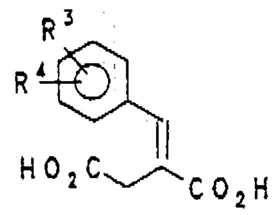

(4)

in das Anhydrid der Formel (7) überführt, wobei $R^3$ und $R^4$ die obengenannte Bedeutung haben

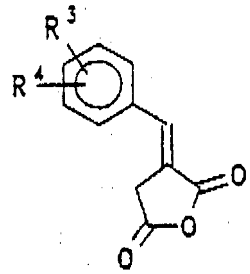

(7)

b) dieses Anhydrid der Formel (7) in einer regioselektiven Reaktion mit einem Amin der Formel $R^1R^2NH$ zum Monoamid der Formel (8) umsetzt, wobei $R^1$, $R^2$, $R^3$ und $R^4$ die obengenannten Bedeutungen haben, und

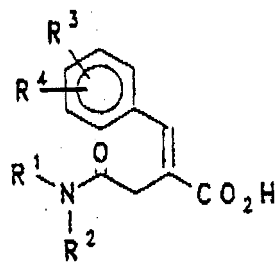

(8)

c) dieses Monoamid der Formel (8) in Gegenwart eines chiralen Rhodium(I)- oder Ruthenium(II)-Diphosphin-Katalysators, dessen Chiralität nicht ausschließlich auf der Anwesenheit chiraler Phosphor-Atome beruht, asymmetrisch zum 2(R)-Benzylbernsteinsäure-Derivat (1) hydriert.

Bevorzugt hat die Gruppierung $NR^1R^2$ die folgenden Bedeutungen:

- Pyrrolidinyl
- Pyrazolidinyl
- Imidazolidinyl
- Piperidinyl
- Piperazinyl
- Tetrahydropyrimidinyl
- 3-Hydroxypiperidinyl
- 4-Hydroxypiperidinyl
- 3-Aminopiperidinyl
- 4-Aminopiperidinyl
- Morpholinyl
- 1,4-Diazacycloheptyl (Homopiperazinyl)

(Hydroxy- und Aminofunktionen jeweils geschützt oder ungeschützt)
$R^3$, $R^4$, $R^5$ stehen für Wasserstoff.

Als Schutzgruppen für Aminogruppen bzw. Hydroxyfunktionen kommen die dem Fachmann allgemein bekannten Strukturen in Frage.

Das erfindungsgemäße Verfahren beruht auf der asymmetrischen Hydrierung einer α,β-ungesattigten Carbonsäure, das Verfahren ist prinzipiell auch im technischen Maßstab durchführbar.

Im Schritt 1 des ersten Verfahrens (Schema 1) wird aus einem Bernsteinsäurediester 2 ($R^6$ entspricht einem geradkettigen oder verzweigten Alkylrest) und einem Benzaldehyd 3 in Gegenwart einer Base im Zuge einer Stobbe-Kondensation ein intermediärer Halbester gewonnen, der ohne Aufarbeitung oder Isolierung durch Verseitung in die Phenylitaconsäure 4 umgewandelt werden kann, wobei dieser Schritt literaturbekannt ist (W.S. Johnson, Organic Reactions Bd. 6, Seiten 1 bis 73 (1951). Die Reaktion kann im Prinzip in einer großen Anzahl von Lösungsmitteln unter Verwendung verschiedener Basen in einem breiten Temperaturinterval durchgeführt werden (siehe 1. Johnson, s.o.; 2. E.C. Horning, J. Am. Chem. Soc. Bd. 74, 5147 (1952); 3. A.M. El-Abbady et al., J. Org. Chem. Bd. 26, 4871 (1961); 4. L.S. El-Assal et al., J. Chem. Soc. 2983 (1963); 4. K.R. Rao, Indian J. Chem. Bd. 7, 859 (1969); 6. J. Andersson et al., Nouv. J. Chim. Bd. 1, 413 (1977); 7. T. Momose et al., Chem. Pharm. Bull. Bd. 25, 2755 (1977)).

**Schema 1:** **Verfahrensvariante 1**

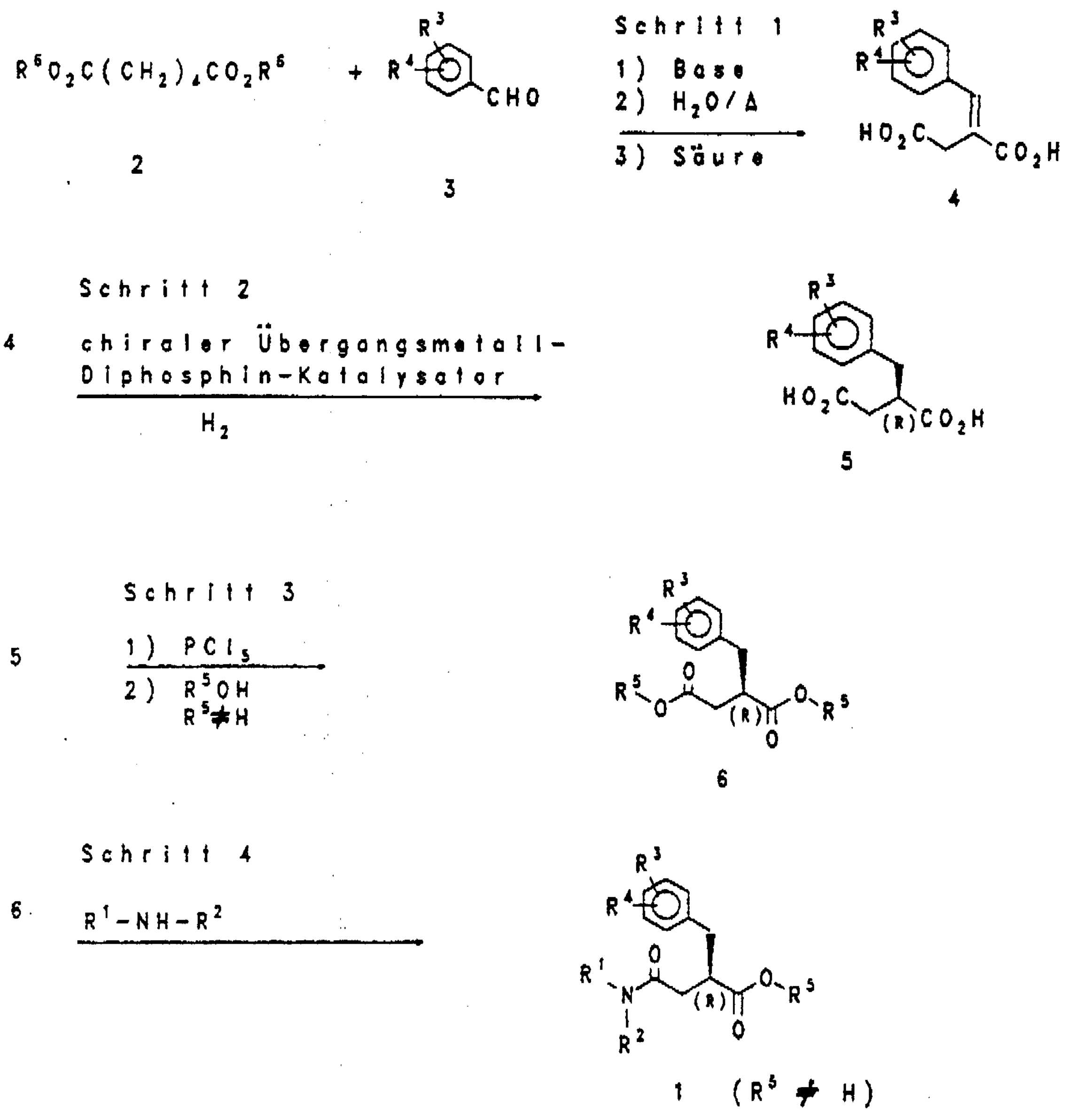

Schema 2: Erfindungsgemäße Verfahrensvariante

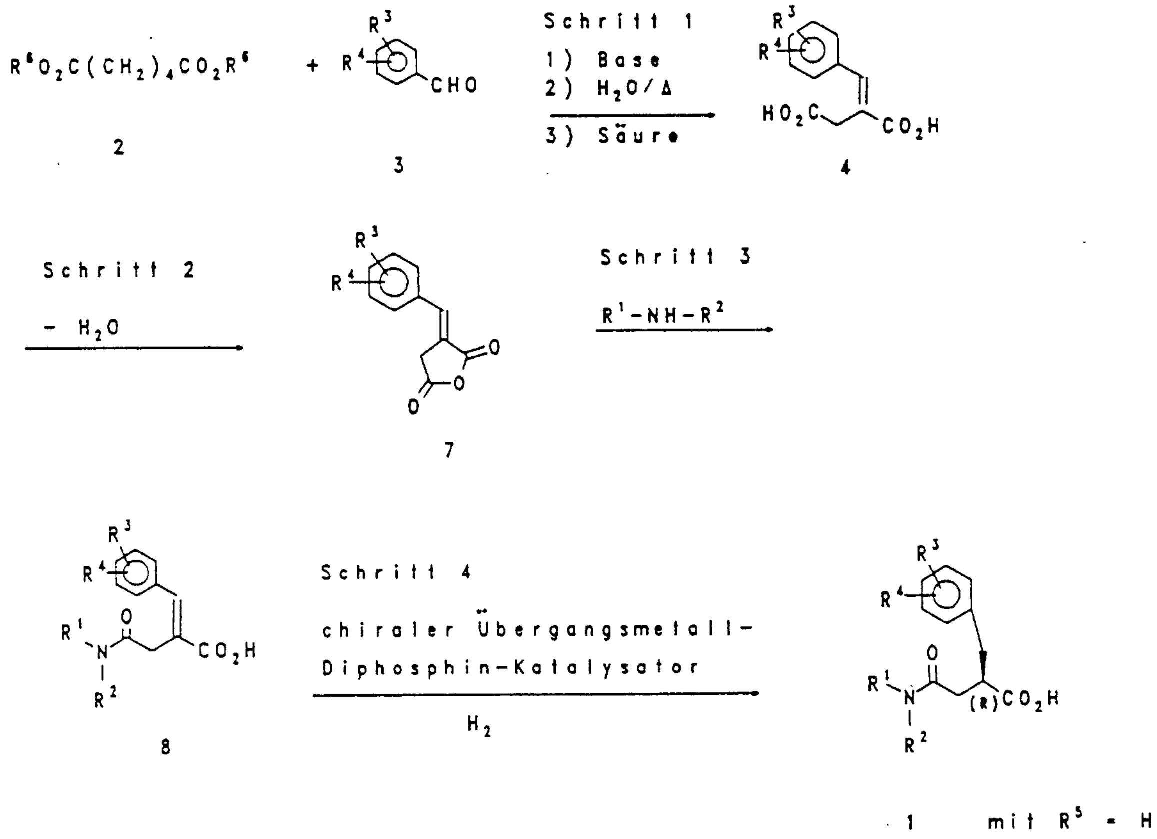


2 3 4 7 8

1 mit $R^5$ = H

Es ist bekannt, daß Verbindungen der allgemeinen Formel 4 alternativ auch durch Wittig-Reaktion von 2-Triphenylphosphoranyliden-bernsteinsäurehalbester mit Benzaldehyd 3 (A. Maercker, Org. React. 14, 270 - 490 (1965)) gefolgt von Verseifung des Halbesters von 4, hergestellt werden können. Es ist weiterhin bekannt, daß Verbindungen der allgemeinen Formel 4 alternativ auch durch eine sogenannte Heck-Reaktion gewonnen werden können (R.F. Heck, Palladium Reagents in Organic Syntheses, Academic Press, New York, 1985), indem die Itaconsäure oder ihr Halb- oder Diester in Gegenwart katalytischer Mengen eines Palladiumsalzes, z. B. $Pd(OAc)_2$ und Triphenylphosphin, gegebenenfalls in Gegenwart einer Base wie Triethylamin, mit einem Halogenbenzol $R^3R^4$-$C_6H_3$-X (X = Cl, Br, I, bevorzugt I) gekuppelt wird und gegebenenfalls der entstehende Halb- oder Diester anschließend zur Dicarbonsäure 4 verseift wird.

Im Schritt 2 des ersten Verfahrens (Schema 1) wird die Phenylitaconsäure 4 asymmetrisch zur optisch aktiven (R)-Benzylbernsteinsäure 5 hydriert. Es ist bekannt, daß im Prinzip heterogene enantioselektive Hydrierungen mit einem Enantiomerenüberschuß von > 90 % ee an Raney-Nickel-Katalysatoren erzielt werden können, die mit Weinsäure und Natriumbromid modifiziert wurden (T. Harada et. al. Chem. Lett. 1195 (1978); A.Tai et. al. Chem. Lett. 2083 (1984)). Wegen des bekannten allergenen Potentials von Nickel und der schlechten Reproduzierbarkeit der Methode erscheinen derartige heterogene Katalysatoren beispielsweise für die Synthese von Pharmaka wenig attraktiv.

Die optische Induktion wird in der vorliegenden Anmeldung darum unter Verwendung eines gelösten, optisch reinen, metallorganischen Komplexes als Hydrierkatalysator erzielt. Der relativ teure Katalysator braucht nur in sehr niedriger Konzentration im Reaktionsgemisch vorhanden zu sein. Die molaren Verhältnisse von umgesetztem Substrat zu eingesetztem Katalysator liegen im allgemeinen je nach Katalysator und Substrat zwischen 100 und ca. 50.000, vorzugsweise zwischen 100 und 5.000. Die obere Grenze des Substrat/Katalysator-Verhältnisses ist im wesentlichen dadurch bedingt, daß der unter Hydrierbedingungen vorliegende "aktive Katalysator" in der Regel durch Sauerstoff rasch irreversibel verbraucht wird. Die benötigte Katalysatormenge hängt deshalb wesentlich davon ab, wie gründlich die Reaktionslösung vor Hydrierbeginn entgast werden kann und wie vollständig der Luftausschluß während der laufenden Hydrierung ist [siehe z. B. W. Vocke et. al. Chem. Techn. 39, 123 (1987)].

In der Regel sind 0,1 - 0,5 Mol-% Katalysator ausreichend. Es kommen vorzugsweise Komplexe zur Anwendung, die als Zentralatom entweder ein Rhodium(I)-Kation (siehe 11, 13 in Schema 3) oder ein Ruthenium(II)-Kation (siehe 16, 18, 19) aufweisen.

Vor Hydrierbeginn liegt eine Lösung des Substrats (4 oder 8), des Präkatalysators und gegebenenfalls von Additiven (z. B. eines Amins) in einem geeigneten inerten Lösungsmittel bzw. Lösungsmittelgemisch vor. Die entgaste Lösung wird dann unter 1 x $10^5$ bis 203 x $10^5$ Pa (1 bis 200 atm) bevorzugt unter 1 x $10^5$ bis 51 x $10^5$ Pa (1 bis 50 atm) Wasserstoffgas geschüttelt oder gerührt.

Anstelle von Wasserstoffgas kann auch eine Transferhydrierung in Gegenwart einer Wasserstoffquelle, z. B. Ameisensäure/Triethylamin oder Isopropanol, zur Anwendung kommen.
Das Prinzip der enantioselektiven Transferhydrierung von C=C-Doppelbindungen wurde beschrieben von H. Brunner et al. [J. Organomet. Chem. 387, 209 (1990)]. Anwendungen dieses Prinzips zur asymmetrischen Hydrierung von 4 oder 8 finden sich unter den Beispielen der vorliegenden Anmeldung.

Die Hydrierung kann in Abhängigkeit von Substrat und Katalysator im Temperaturbereich von -40°C bis +80°C, bevorzugt bei -20 bis +40°C, besonders bevorzugt bei 0 bis 30°C durchgeführt werden. Man vermutet, daß der sauerstoffempfindliche "aktive Katalysator" durch reduktive Abspaltung der Liganden $Y^1$ (im Falle von 11) bzw. L---L (im Falle von 13) entsteht und die Struktur 14 besitzt, in dem die beiden freigewordenen Koordinationsstellen vom Substrat (z. B. den beiden Carboxylatfunktionen im Falle einer Hydrierung von 4 in Gegenwart von Triethylamin) besetzt werden. Der Umfang der Hydrierung (von 4 zu 5 bzw. von 8 zu 9) läßt sich durch Messung des Wasserstoffverbrauchs oder durch HPLC-Analyse verfolgen. Die Enantioselektivität der Hydrierung eines gegebenen Substrats wird von Parametern wie Reaktionstemperatur, Wasserstoffdruck, Natur des Lösungsmittels, Anwesenheit von Additiven in z. T. erheblichem Maße beeinflußt. Diese Effekte sind im allgemeinen schwer vorhersagbar. Zum Beispiel geht die Enantioselektivität einiger optisch aktiver Rhodium(I)-diphosphin-Komplexe deutlich zurück, wenn ein bestimmter Wasserstoffgrenzdruck überschritten wird, während beim Vorliegen eines andersartigen Diphosphins der Enantiomerenüberschuß (ee) des Produkts entweder unbeeinflußt bleibt oder sogar zunimmt. In der Regel nimmt die Hydriergeschwindigkeit mit steigendem Wasserstoffdruck zu und man benötigt weniger Katalysator. Auch eine Temperaturerhöhung führt in der Regel zu einer Beschleunigung der Hydrierung, ist jedoch häufig mit einer Erniedrigung des Enantiomerenüberschusses und einer geringeren Lebensdauer des Katalysators verbunden. In manchen Fällen wird jedoch das optimale Ergebnis durch Temperaturerhöhung und Hydrierzeitverkürzung erreicht. In der Regel wird man die Enantioselektivität der Hydrierung eines gegebenen Substrat/Katalysator-Paares durch Kühlung auf - 20°C bis 0°C unter Inkaufnahme einer längeren Hydrierdauer verbessern können. Schwer berechenbar, aber z. T. wichtig, ist der Einfluß eines Additivs, wie z. B. Triethylamin oder optisch aktives 1-Phenylethylamin, auf die Enantioselektivität der asymmetrischen Hydrierung. Während z. B. die Hydrierung von Phenylitaconsäuren 4 mit dem Rhodiumkomplex von BPPM (20 bzw. 21) nur in Anwesenheit eines Äquivalents eines Amins zu Benzylbernsteinsäuren 5 mit hohem Enantiomerenüberschuß führt, bleibt bei einigen anderen Substrat/Katalysator-Paaren die Enantioselektivität in Gegenwart von Triethylamin unbeeinflußt oder nimmt in wenigen Fällen sogar stark ab.

Ebenfalls schwer erfaßbar ist für ein gegebenes Metall ($Rh^I$ oder $Ru^{II}$) und ein gegebenes optisch aktives Diphosphin die Überlegenheit oder Unterlegenheit eines kationischen Präkatalysators gegenüber einem neutralen Präkatalysator (siehe Schema 3). In der Mehrzahl der Fälle haben die kationischen Rhodium(I)-komplexe 13 eine höhere katalytische Aktivität und eine größere Enantioselektivität als entsprechende neutrale Komplexe 11. In einigen Fällen liegen jedoch entgegengesetzte Verhältnisse vor.

Als Lösungsmittel für die asymmetrische Hydrierung sind im Prinzip alle Flüssigkeiten geeignet, die Substrat, Katalysator und eventuell zugesetzte Additive lösen und unter den Reaktionsbedingungen inert sind. Diese Voraussetzungen werden u. a. von unverzweigten und verzweigten Alkoholen erfüllt. Wegen der leichten Entfernbarkeit im Vakuum werden dabei Methanol, Ethanol, Propanol oder Isopropanol bevorzugt. Einige Katalysatoren zeigen jedoch eine mit fallender Lösungsmittelpolarität zunehmende Enantioselektivität, ergeben also in Isopropanol verglichen mit Methanol Produkte mit höherem Enantiomerenüberschuß. In diesen Fällen ist es zweckmäßig, ein mit dem Alkohol mischbares unpolares Co-Solvenz zuzusetzen bzw. die Hydrierung direkt in einem wenig polaren Lösungsmittel durchzuführen.

In Schema 3 ist die Herstellung der optisch aktiven Präkatalysatoren skizziert, Schema 4 ist eine Zusammenstellung einiger optisch aktiver Bis(diarylphosphino)-Verbindungen der allgemeinen Formel 10, die in der vorliegenden Anmeldung untersucht wurden. Von diesen sind die Verbindungen 20, 24 - 27, 29 und 33 kommerziell erhältlich; die Verbindungen 21 (G.L. Baker et.al., J. Org. Chem. 46, 2954 (1981)), 22 (I. Ojima et. al., Tetrahedron Lett. 21, 1051 (1980)) und 28 (H.W. Krause et. al. DD-A 253 947) wurden nach Literaturmethoden hergestellt. Die Verbindungen 23, 30, 31, 32 sind nicht vorbeschrieben, ihre Herstellung ist in den Beispielen der vorliegenden Anmeldung beschrieben. Es gibt einige weitere kommerziell erhältliche und eine Vielzahl weiterer literaturbekannter chiraler Diphosphine der allgemeinen Formel 10. Die Verwendung aller dieser Diphosphine 10 sowie weiterer Diphosphine der allgemeinen Formel 10 im Rahmen des hier beschriebenen Verfahrens zur Herstellung von Verbindungen der allgemeinen Formel 1, gehört zu der vorliegenden Erfindung.

Eine Übersicht über bekannte chirale Diphosphine der allgemeinen Formel 10 und deren Verwendung zur asymmetrischen Hydrierung von C=C-Doppelbindungen erhält man bei:

a) K.E. Koenig in "Asymmetric Synthesis", Vol. 5, Ed. Morrison, J.D. Academic Press, Orlando 1985, Seite 71ff;
b) J.W. ApSimon et.al., Tetrahedron 42, 5157 (1986), Seite 5173 - 5186;
c) H. Brunner, Top. Stereochem. 18, 129 (1988);
d) I. Ojima et. al., Tetrahedron 45, 6901 (1989), Seite 6902 - 6916.

**Schema 3:** Chirale Übergangsmetall-Diphosphin-Komplexe zur Durchführung der katalytischen asymmetrischen Hydrierungen von Dicarbonsäure 4 bzw. Monocarbonsäuremonoamid 8 (vgl. Schemata 1 und 2).

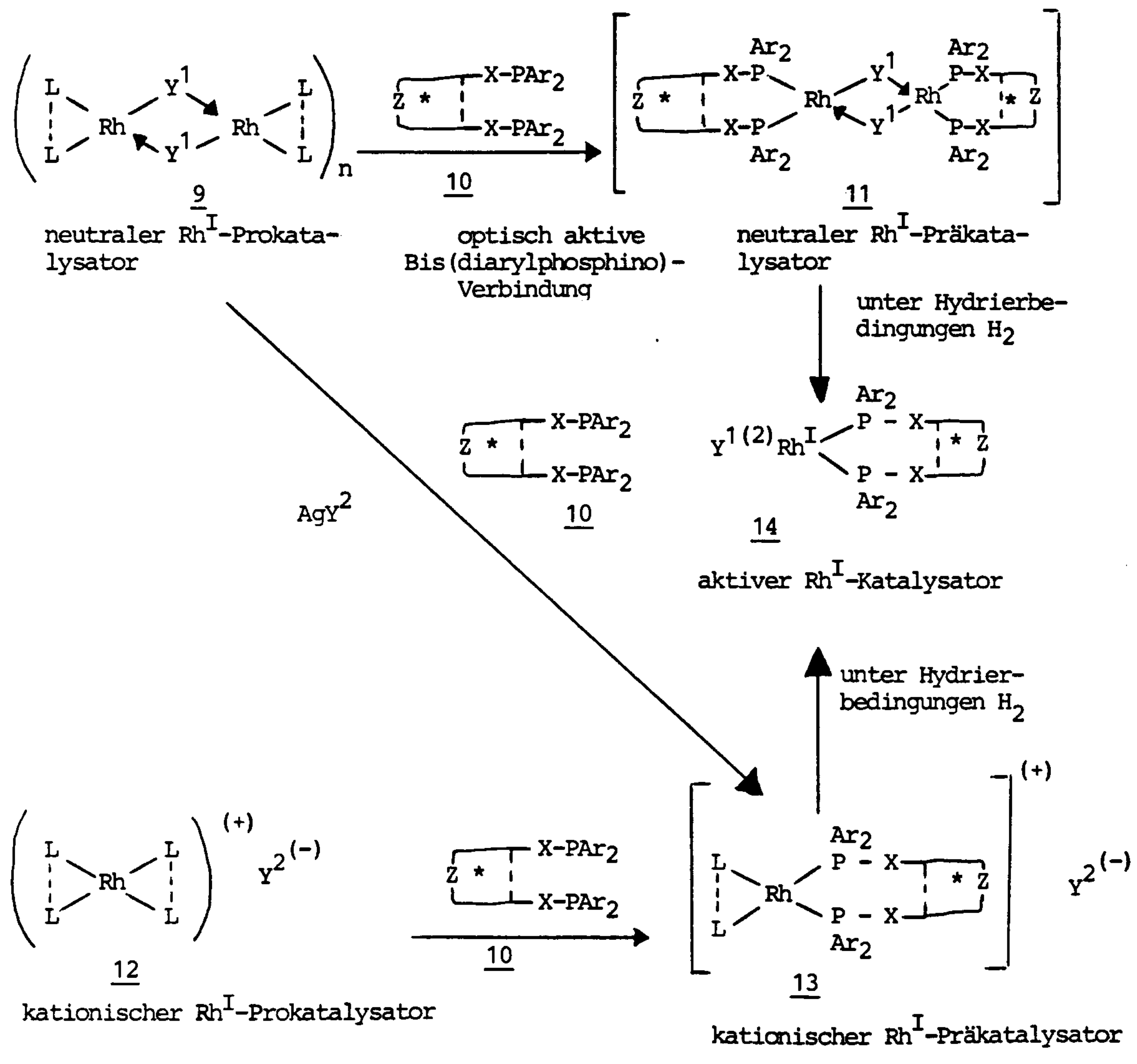

Schema 3 Fortsetzung

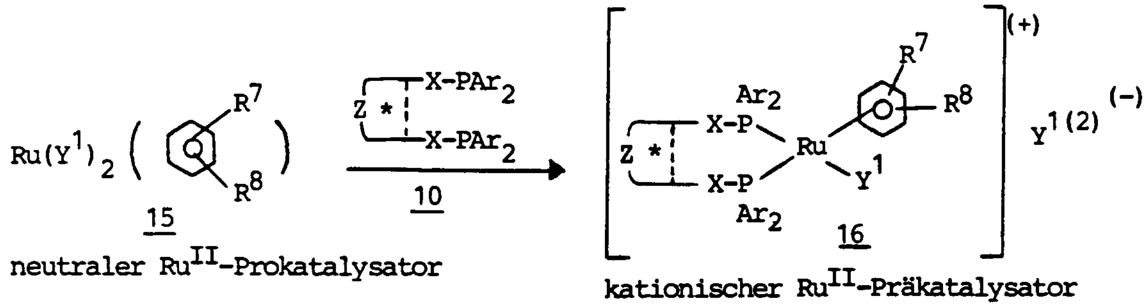


15 neutraler $Ru^{II}$-Prokatalysator

16 kationischer $Ru^{II}$-Präkatalysator

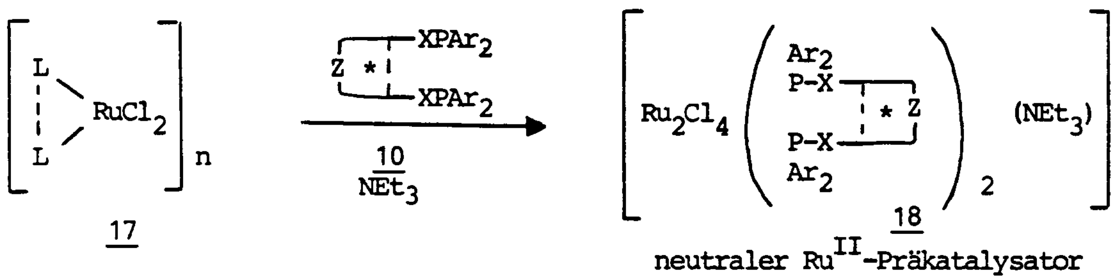


17

18 neutraler $Ru^{II}$-Präkatalysator

18

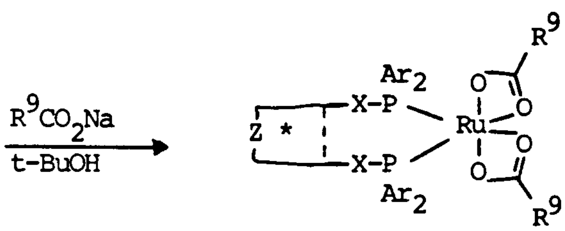


19 neutraler $Ru^{II}$-Präkatalysator

Schema 4: In den Beispielen der vorliegenden Erfindung angewandte optisch aktive Bis(diarylphosphino)-Verbindungen der allgemeinen Formel:

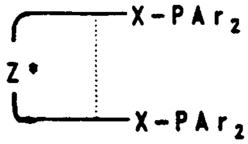 :

10

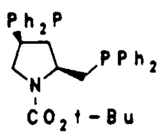

(2S,4S)(-)-BPPM

20

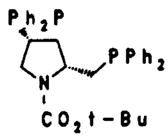

(2R,4R)(+)-BPPM

21

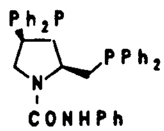

(2S,4S)(-)-Phenyl-CAPP

22

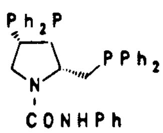

(2R,4R)(+)-Phenyl-CAPP

23

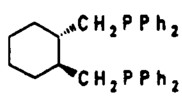

24

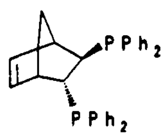

(-)-Norphos

25

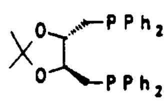

(+)-DIOP

26

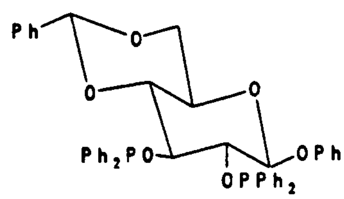

(-)-Phenyl-4,6-O-(R)-benzyliden-2,3-(O)-bis-(diphenylphosphino)-β-D-glucopyranosid
Phenyl-β-glup 27

## Schema 4 Fortsetzung

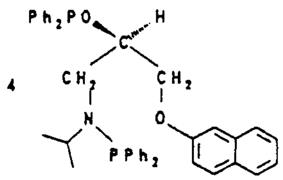

(R)(-)Propraphos
PPP

28

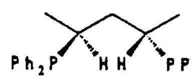

(2S,4S)(-)-Bis-(diphenylphosphino)pentan
BDPP

29

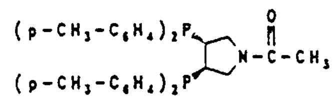

(3R,4R)(+)-1-Acetyl-3,4-bis(di-p-tolylphosphino)-pyrrolidin

30

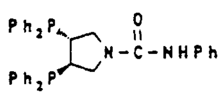

(3R,4R)(+)-1-Phenylamino-carbonyl-3,4-bis(diphenyl-phosphino)-pyrrolidin

31

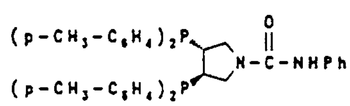

(3R,4R)(+)-1-Phenylamino-carbonyl-3,4-bis(di-p-tolylphosphino)-pyrrolidon

32

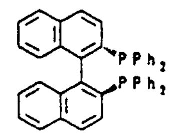

(S)(-)-BINAP

33

Aus den angegebenen Übersichtsartikeln geht auch hervor, daß die chiralen Hydrierkatalysatoren in mannigfaltiger Weise modifiziert, z. B. durch Adsorption oder Bindung (evtl. über Spacer) an anorganischen Feststoffen (Kieselgel, Aktivkohle.) oder an quellfähigen Harzen (Polymeren) immobilisiert (siehe H. Brunner et.al. J. Organomet. Chem. 384, 223 (1990)), oder durch geeignete hochpolare Substituenten in wasserlösliche Katalysatoren überführt werden können. Immobilisierte Katalysatoren können nach Reaktionsende durch Filtration aus dem Reaktionsgemisch entfernt werden, was eine Vereinfachung der Aufarbeitung darstellt. Außerdem können die abfiltrierten Katalysatoren gelegentlich ohne Aktivitäts- oder Enantioselektivitätsverlust in späteren Hydrierchargen wiederholt eingesetzt werden (Recycling). Der Einsatz wasserlöslicher Katalysatoren erlaubt die umweltfreundliche und billige Anwendung von Wasser als Lösungsmittel bzw. die einfache Entfernung des Katalysators nach Hydrierende durch Waschen mit Wasser bei Verwendung eines unpolaren Lösungsmittels.

Experimentell zeigt sich weiterhin, daß die Racematspaltung von rac.-1 ($R^5$ = H) mit schlechter Ausbeute verläuft. Verwendet man hingegen (R)-1 ($R^5$ = H) von $\geq$ 80 % ee, so wird optisch reines (R)-1 in fast quantitativer Ausbeute mit einer einzigen Kristallisation erhalten.

Die Möglichkeit des Anschlusses eines derartigen Racematspaltungsschrittes zur Erhöhung der optischen Reinheit von Verbindungen der Formel 1 gehört deshalb auch zum vorliegenden Verfahren zur enantioselektiven Synthese von Verbindungen der allgemeinen Formel 1.

In Schema 3 haben die Symbole die folgenden Bedeutungen:

L ist ein monodentater (in der Regel achiraler) Ligand, der eine Koordinationsstelle in der Ligandensphäre von $Rh^{I}$ oder $Ru^{II}$ besetzt. Es ist vorteilhaft, wenn zwei dieser monodentaten Liganden L unter Ausbildung eines Rings miteinander verbunden sind. Diese Verhältnisse liegen bei einem cyclischen bidentaten Liganden vor. Eine bevorzugte Bedeutung von L---L ist darum z. B. 1,5-Cyclooctadien oder 2,5-Norbornadien. $Y^1$ hat die Bedeutung Chlor, Brom oder Jod, bevorzugt Chlor. $Y^2$ ist ein Anion, das weniger nucleophil als Chlorid ist, also z. B. $BF_4^-$, $SbF_4^-$, $SbF_6^-$, $PF_6^-$, $ClO_4^-$, $CF_3SO_3^-$, bevorzugt $BF_4^-$. Im aktiven Katalysator 14 kann das Anion Y sowohl die Bedeutung von $Y^1$ als auch $Y^2$ haben, je nachdem ob der neutrale Präkatalysator 11 oder der kationische Präkatalysator 13 vorgeschaltet war. Ebenso kann im kationischen $Ru^{II}$-Komplex 16 das Anion Y sowohl die Bedeutung von $Y^1$ als auch $Y^2$ haben. $R^7$ und $R^8$ sind gleich oder verschieden und haben die Bedeutung von Wasserstoff oder geradkettigem oder verzweigtem Alkyl mit 1 - 4 Kohlenstoffatomen. $R^9$ hat die Bedeutung von geradkettigem oder verzweigtem Alkyl oder Alkenyl mit 1 - 4 Kohlenstoffatomen. Die optisch aktiven Bis(diarylphosphino)-Verbindungen der allgemeinen Formel 10 umfassen, wie man den Beispielen des Schemas 4 entnimmt, eine Vielzahl von Strukturtypen. Die Chiralität ist dabei stets im Kohlenstoff-Gerüst begründet, was durch den Stern im entsprechenden Molekülteil symbolisiert wurde. Außerhalb des strukturellen Geltungsbereichs der allgemeinen Formel 10 liegen solche Diphosphine, deren Chiralität ausschließlich auf der Anwesenheit

chiraler Phosphor-Atome beruht, die also die allgemeine Formel

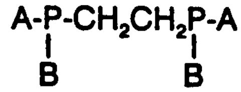

aufweisen.

Die chirale Phosphorverbindung DIPAMP (M.S. Knowles et.al., DE 2 456 937) und dessen Analoga werden somit nicht von der vorliegenden Erfindung umfaßt. Die Verbindungen der allgemeinen Formel 10 können ein offenkettiges (siehe z. B. 28, 29 und 33 in Schema 4) oder ein mono- oder polycyclisches (siehe z. B. 24, 25) chirales Kohlenstoffgerüst haben. Dieser Umstand wurde in 10 durch die gestrichelte Linie symbolisiert. Das chirale Kohlenstoffgerüst kann ein oder mehrere Heteroatome Z beeinhalten, die die Bedeutung Stickstoff N oder Sauerstoff O haben können [siehe z. B. 20 - 23, 26, 27). Die beiden Diarylphosphinogruppen können über Kohlenstoffatome X = $CH_2$ (siehe z. B. 26) oder über Heteroatome X (X = O oder N) mit dem chiralen Kohlenstoffgerüst verbunden sein [siehe z. B. 28, 27], die Diarylphosphinogruppen können aber auch direkt an das chirale Kohlenstoffgerüst gebunden sein (in diesen Fällen symbolisiert X kein Atom, sondern eine chemische Bindung; siehe z. B. 24, 25, 29 - 33).

Die literaturbekannten asymmetrischen Hydrierungen von C=C-Doppelbindungen, die von den vier oben zitierten Review-Arbeiten erfaßt sind, gelten weitgehend Hydrierungen von N-Acyl-dehydroaminosäuren. Diese Arbeiten sind bezüglich der vorliegenden Erfindung nicht relevant, da die Durchführbarkeit und die Enantioselektivität asymmetrischer Hydrierungen bekanntlich in hohem Maße von der Natur und der relativen räumlichen Anordnung von koordinationsfähigen funktionellen Gruppen des Substrats zu der zu hydrierenden Doppelbindung abhängen. Asymmetrische Hydrierungen von Substraten, die strukturell den hier relevanten allgemeinen Formeln 4 und 8 ähneln, werden z. B. von K.E. Koenig (in "Asymmetric Synthesis" Vol. 5, Seite 90 in Tab. 11) beschrieben, der die bekannten asymmetrischen Hydrierungen von Itaconsäure zusammenfaßt. Die Itaconsäure unterscheidet sich von der allgemeinen Formel 4 dadurch, daß sie nicht den Substituenten $R^3R^4C_6H_3$- besitzt. Man entnimmt dieser Publikation, daß die 2(S)-Methylbernsteinsäure mittels des kommerziell verfügbaren chiralen Diphosphins (S,S)-BPPM 20 mit guter Enantioselektivität zugänglich war. Die im Zusammenhang mit der vorliegenden Erfindung relevante (R)-Konfiguration war jedoch nicht befriedigend zugänglich. Über asymmetrische Hydrierungen von Itaconsäuremonoamiden liegen kaum Erfahrungen vor. Hier ist nur die Hydrierung eines Mono-Benzylamids der Itaconsäure mit (R,R)-DIPAMP (siehe K.E. Koenig, Tab. 11, Seite 91) bekannt. Die zugrundeliegende Originalarbeit (W.C. Christopfel, B.D. Vineyard, J. Am. Chem. Soc. 101, 4406 (1979) beschreibt nicht die vorliegende Erfindung, da

a) dort nicht mit Phenylitaconsäurederivaten gearbeitet wurde,
b) das Katalysator-Diphosphin DIPAMP außerhalb der Definitionen der vorliegenden Erfindung liegt (vide supra).

In Schritt 3 des ersten Verfahrens (Schema 1) wird die optisch aktive Benzylbernsteinsäure in einen Diester 6 umgewandelt. Der Diester 6 kann im Prinzip durch direkte Veresterung der Dicarbonsäure 5 mit der Hydroxyverbindung $R^5OH$ oder durch intermediäre Bildung eines doppelten Säurehalogenids hergestellt werden. Das Säurehalogenid kann im Prinzip mittels Phosphorpentachlorid Phosphortrichlorid, Phosphoroxychlorid, Phosphortribromid, Thionylchlorid, Oxalylchlorid oder anderer Reagenzien, die zur Erzeugung von Carbonsäurehalogeniden aus Carbonsäuren befähigt sind, hergestellt werden. Das intermediäre Säurehalogenid kann, z. B. durch Destillation, isoliert werden, oder aber ohne Isolierung direkt in den Diester 6 umgewandelt werden.

Bevorzugt wird die intermediäre Herstellung des doppelten Säurechlorids mittels Phosphorpentachlorid in Toluol bei 40° - 45°C, Entfernung des Lösungsmittels im Vakuum und direkte Umsetzung des öligen Rückstands mit der Hydroxyverbindung $R^5OH$. Im Prinzip lassen sich so Diester 6 herstellen, deren Rest $R^5$ nur von der Verfügbarkeit der Hydroxyverbindung $R^5OH$ limitiert ist. In der Praxis hängt die Qualität des Verfahrens aber entscheidend davon ab, daß der Diester 6 und damit der Rest $R^5$ verschiedenen Vorgaben genügt. Diese sind:

a) Der Diester 6 muß unter den Bedingungen seiner Herstellung und seiner Umsetzung zum Monoamid 1 ($R^5 \neq H$) stabil gegen Racemisierung sein.
b) Der Diester 6 darf nicht zu stark aktiviert sein, da sich sonst bei der Umsetzung zum Monoamid 1 ($R^5 \neq H$) keine ausreichende Regioselektivität einstellt.
c) Der Diester 6 darf andererseits aber auch nicht zu stark desaktiviert sein, da das Monoamid 1 ($R^5 \neq H$) z. B. zur Herstellung von Reninhemmern racemisierungsfrei kupplungsfähig mit primären oder sekundären Aminogruppen sein soll. Zwar existiert gemäß DE-A 39 30 397 eine bekannte Kupplungsmethode derartiger Amine und der freien Carbonsäure 1 ($R^5 = H$), die Verseifung von Estern der Formel 1 ($R^5 \neq H$) zu Carbonsäuren der Formel 1 ist jedoch unter den klassischen Bedingungen (verdünnte wäßrige Natronlauge) mit Racemisierung verbunden (siehe Beispiele). Es ist aus diesem Grunde sowie zur Einsparung von Syntheseschritten notwendig, einen direkt kupplungs-

fähigen Ester 1 ($R^5 \neq H$) zu erhalten.

d) Die Hydroxyverbindung $R^5OH$ soll einfach zugänglich bzw. preiswert sein.

e) Der Diester 6 soll zwecks bequemer Reinigung eine hohe Kristallisationstendenz haben.

Es wurde gefunden, daß substituierte Phenylverbindungen und insbesondere die Hydroxyverbindung p-Nitrophenol ($R^5 = O_2N\text{-}C_6H_4\text{-}$) die Vorgaben a) bis e) befriedigend erfüllt. Beispielsweise ist die Reaktion des rohen doppelten Säurechlorids mit 2,05 Mol-Äquiv. p-Nitrophenol in Gegenwart von 2,5 Mol-Äquiv. Triethylamin bei -20 bis 80°C, bevorzugt bei 15 bis 25°C in geeigneten inerten Lösungsmitteln unter den bevorzugten Bedingungen innerhalb 8 - 24 Std. quantitativ. Die Bis-(p-nitrophenyl)ester 6 ($R^5 = p\text{-}O_2NC_6H_4$) haben eine hohe Kristallisationstendenz und sind aus konzentrierter organischer Lösung leicht durch Zugabe von z. B. Kohlenwasserstoffen oder unpolaren Ethern, bevorzugt Diisopropylether oder Methyl-tert.-butyl-ether, nahezu quantitativ ausfällbar. Die umgefällten Diester 6 sind chemisch rein. Ihre optische Reinheit entspricht mindestens der der eingesetzten Dicarbonsäuren 5. Ausgehend von Dicarbonsäuren 5 hoher optischer Reinheit (≥ 95 % ee) erhält man praktisch optisch reine Diester 6 ($R^5 = p\text{-}O_2N\text{-}C_6H_4\text{-}$, >99 % ee).

In Schritt 4 des ersten Verfahrens (Schema 1) wird der optisch aktive (optisch reine) Diester 6 mit dem Amin $R^1R^2NH$ zum Monoamid 1 umgesetzt. Die Reaktion kann im Prinzip in einem beliebigen inerten Lösungsmittel und in einem weiten Temperaturinterval (z. B. -40°C bis + 100°C) durchgeführt werden. Die Umsetzung des bevorzugten Bis(p-nitrophenyl)esters ($R^3$,$R^4$ = H, $R^5 = p\text{-}O_2N\text{-}C_6H_4\text{-}$) mit den bevorzugten Aminen $R^1R^2NH$ verläuft, z. B. in DMF bei -10°C bis 30°C in 0,5 - 5 Std. quantitativ. Das entstehende p-Nitrophenol kann aus organischer Lösung des Rohprodukts mit 0,5 molarer Natronlauge bequem quantitativ extraktiv entfernt werden. Es fällt beim Ansäuern der wäßrigen Extrakte aus und kann nach dem Absaugen und Trocknen zur Herstellung späterer Chargen 6 aus 5 wiederverwendet werden (Reagenz-Recycling). Das aufgearbeitete Rohprodukt 1 weist bereits eine hohe Regioselektivität auf.

Die Umsetzung des gemäß Schema 1 gewonnenen Aktivestermonoamids 1 mit primären oder sekundären Aminen zu entsprechenden Diamiden, z. B. Reninhemmern, verläuft unter milden Bedingungen in guter Ausbeute ohne Racemisierung.

Die bevorzugten Reaktionsbedingungen für die Reaktion des Monoesters 1 zum Diamid orientieren sich an den Bedingungen für die Umsetzung des Diesters 6 zum Monoamid 1. Die bevorzugte Reaktionstemperatur ist mit 20 - 70°C, insbesondere 30 - 50°C etwas höher als bei der Umsetzung des Diesters 6.

Das erfindungsgemäße Verfahren ist in Schema 2 skizziert. Der Schritt 1 dieses Verfahrens ist identisch mit dem entsprechenden Schritt des ersten erfindungsgemäßen Verfahrens.

Der Schritt 2 des erfindungsgemäßen Verfahrens ist eine Kondensation der ungesättigten Dicarbonsäure 4 unter Abspaltung von Wasser und Bildung des ungesättigten Anhydrids 7. Diese Reaktion kann mit einer Vielzahl von wasserabspaltenden Reagenzien oder rein thermisch durchgeführt werden (siehe z. B. T. Eicher et al. in "Houben-Weyl, Methoden der Organischen Chemie", Bd. E5 "Carbonsäuren und Carbonsäure-Derivate", J. Falbe Edit., Seiten 633 - 652: "Carbonsäureanhydride", G. Thieme Verlag, Stuttgart 1985).

Lösungsmittel und Reaktionstemperatur sind unkritisch. Man kann die Reaktion auch ohne Lösungsmittel durchführen, wobei das wasserabspaltende Reagenz entweder flüssig ist und partielle Lösung der Dicarbonsäure bewirkt oder aber als Festphasenreaktion. Bevorzugt wird die Durchführung der Reaktion bei Raumtemperatur in einem geeigneten Ether, z. B. THF, in Gegenwart eines Überschusses von Acetanhydrid.

Im Schritt 3 des erfindungsgemäßen Verfahrens wird das Anhydrid 7 mit dem Amin $R^1R^2NH$ regioselektiv unter Bildung des ungesättigten Monoamids 8 geöffnet $R^1$ und $R^2$ haben die vorstehend gegebenen Definitionen. Die Reaktion kann im Prinzip in einem beliebigen inerten Lösungsmittel und in einem weiten Temperaturinterval (z. B. -40°C bis +100°C) durchgeführt werden. Für gegebene Reaktanden, Lösungsmittel und Reaktionstemperatur kann die Regioselektivität signifikant erhöht werden, wenn das Amin über einen längeren Zeitraum zur Suspension des Anhydrids 7 zuportioniert wird. Die Umsetzung von 7 mit den bevorzugten Aminen $R^1R^2NH$ kann in polaren und apolaren Lösungsmitteln durchgeführt werden, vorzugsweise im Lösungsmittel Ethylacetat bei 0 - 30°C. Die Reaktion verläuft nach 4 Std. bis 2 Tagen in hoher Ausbeute. Eine HPLC-Trennung der regioisomeren ungesättigten Monoamide des Rohprodukts ist möglich. Hydriert man das Rohprodukt direkt in methanolischer Lösung über Palladium/Kohle, so erhält man das racemische gesättigte Monoamid der Formel 1 und dessen Regioisomer, die sich per HPLC leicht trennen lassen. Auf diese Weise läßt sich das Verhältnis der Regioisomeren im rohen ungesättigten Monoamid ermitteln. Nach Kristallisation z. B. aus MTB-Ether läßt sich das Regioisomeren-Verhältnis verbessern.

In Schritt 4 des erfindungsgemäßen Verfahrens wird das ungesättigte Monoamid 8, in Gegenwart eines chiralen Übergangsmetall-Diphosphin-Katalysators einer asymmetrischen Hydrierung zu 1 ($R^5$ = H) unterworfen. Es gelten die unter Schritt 2 des ersten Verfahrens gemachten Anmerkungen sinngemäß.

Das gesättigte Monoamid 1 ($R^5$ = H) kann, wie bereits konkret in DE-A 39 30 397 und DE-A 39 33 096 vorgestellt, mit primären oder sekundären Aminen racemisierungsfrei zu entsprechenden Diamiden, wie z. B. zu Reninhemmern, umgesetzt werden.

Durch die folgenden Beispiele werden die beiden Verfahren (Schemata 1 und 2) weiter illustriert und konkrete

Durchführungsvarianten geschildert. Die Beispiele zu asymmetrischen Hydrierungen wurden in der vorliegenden Erfindung aus apparativen Gründen überwiegend bei Raumtemperatur unter 1 atm Wasserstoff durchgeführt, die erfindungsgemäße Hydrierung läßt sich jedoch ebenso bei höheren Druckwerten und anderen Temperaturen durchführen.

Allgemeine Arbeitstechnik und Analytik

Optische Reinheiten von Chargen 2-Benzylbernsteinsäure 5 aus asymmetrischen Hydrierungen wurden bestätigt durch HPLC-Trennung beider enantiomerer Dimethylester (hergestellt durch Umsetzung von 5 mit einem Überschuß Diazomethan) auf der Chiral-Phase ®CHIRALCEL OC mit n-Hexan/Isopropanol (75 : 25); 0,5 ml/Min., Raumtemperatur, Detektor 220 nm. Das (R)-Isomer ($R^3$ = $R^4$ = H, Dimethylester) wird nach 14,30 Minuten, das entsprechende (S)-Isomer nach 16,46 Min. eluiert.

Optische Reinheiten der Monoamide ($R^5$ = H) aus asymmetrischen Hydrierungen von 8 wurden nach zwei unabhängigen Analysemethoden bestimmt. Bei der ersten Analysemethode wurden Monoamide 1 ($R^5$ = H) mit überschüssigem Diazomethan zum Monomethylestermonoamid umgesetzt und dessen beide Enantiomeren (auf ®CHIRALCEL OC mit n-Hexan/Isopropanol (70 : 30 bis 50 : 50) 1,0 ml/Min., 35°C, Detektor 220 nm) getrennt. Bei der zweiten Analysemethode wurden Monoamide 1 ($R^5$ = H) unter Katalyse mit optisch reinem (S)-(-)-1-Phenylethylamin zu entsprechenden Diamiden 34 umgesetzt.

Wenn die Verbindung 1 ($R^5$ = H) nicht optisch rein ist, so entsteht bei dieser Reaktion ein Gemisch zweier diastereomerer Verbindungen 34, das RS- und das SS-konfigurierte Produkt.

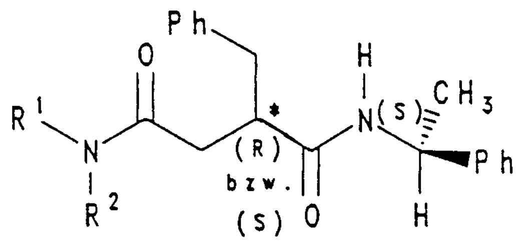

34

Beispiele

(Beispiele 1 bis 3 sind Vergleichsbeispiele)

Beispiel 1, Stufe 1:

(E)-Phenylitaconsäure (Formel 4, $R^3$ = $R^4$ = H)

Zu 21.0 l tert.-Butanol gibt man bei Raumtemperatur unter $N_2$ und unter Rühren 4.0 kg (35.7 Mol, 1.21 Äquiv. bez. auf Benzaldehyd) Kalium-tert.-butylat. Man heizt auf 55°C und rührt so lange, bis eine klare Lösung vorliegt (ca. 30 - 60 Min.). Bei einer Manteltemperatur von O°C kühlt man auf 20°C Innentemperatur ab. Dabei fällt das Kalium-tert.-butylat sehr fein verteilt aus. 6.0 l (36.1 Mol, 1.22 Äquiv. bez. auf Benzaldehyd) auf O°C vorgekühlter Bernsteinsäurediethylester werden innerhalb von 5 Min. zugegossen. Man rührt 5 Min. und gießt dann (weiterhin bei 0°C Manteltemperatur) 3.0 l (3.13 kg, 29.5 Mol, 1.0 Äquiv.) Benzaldehyd innerhalb 5 - 10 Min. zu. Durch die exotherme Reaktion steigt die Innentemperatur auf 32 - 36°C an. Man rührt 20 Min., wobei die Innentemperatur wieder auf 10 - 15°C abfällt. Man läßt 18.0 l 10 %ige wäßrige Natronlauge zulaufen und rührt dann 30 Min. bei einer Innentemperatur von 75°C. Man kühlt auf 40°C ab und trennt die Wasserphase ab. Die Butanol-Phase wird einmal mit 10 l 2 molarer Natronlauge und zweimal mit je 5 l 2 molarer Natronlauge extrahiert. Die vereinigten wäßrigen Phasen (ca. 40 l) werden einmal mit 10 l und einmal mit 5 l Methyl-tert.-butyl-ether gewaschen, dann bei 15 - 20°C mit ca. 5 - 10 l 37 %iger Salzsäure auf pH = 7.5 gestellt. Man läßt 20 l Ethylacetat zu dieser wäßrigen Phase laufen und stellt dann mit weiteren ca. 5 l 37 %iger Salzsäure auf pH = 3.0. Die Wasserphase wird abgetrennt und einmal mit 10 l, dann zweimal mit 5 l Ethylacetat extrahiert. Die vereinigten Essigester-Extrakte werden im Vakuum auf 10 l aufkonzentriert. Man setzt 12 l Butylacetat zu und entfernt das restliche Ethylacetat im Vakuum. Die entstehende Suspension wird eine Stunde bei 10°C gerührt und der

Feststoff abgeschleudert. Er wird mit zweimal 2 l auf 0 - 10°C vorgekühltem Butylacetat, dann mit zweimal 2 l Diisopropylether gewaschen und trockengeschleudert. Das Produkt wird in 15 l Wasser suspendiert, 12 Std. gerührt, abgeschleudert, mit Wasser gewaschen, trockengeschleudert und dann im Vakuum bei 40°C zur Gewichtskonstanz getrocknet.

Man erhält 3.1 kg (15.0 Mol, Ausbeute 51 % d.Th. bez. auf Benzaldehyd) farblosen Feststoff, Schmp. 185 - 188°C. $^1$H-NMR (DMSO-$d_6$): $\delta$ = 12.5 (s,2H,$CO_2$H), 7.74 (s,1H,=C<u>H</u>), 7.20-7.55 (m,5H,arom.-H), 13.38 (s,2H,$CH_2$) ppm.

Stufe 2:

Optisch aktive Benzylbernsteinsäure (Formel 5, $R^3$ = $R^4$ = H) durch asymmetrische Hydrierung von (E)-Phenylitaconsäure

Einen Gesamtüberblick über die durchgeführten asymmetrischen Hydrierungen von (E)-Phenylitaconsäure, die dabei erzielten optischen Induktionen und die zugehörigen Reaktionsparameter gibt Tab. 1. Die Herstellung der neuen chiralen Diphosphine 23, 30, 31, 32, sowie der isolierten kationischen Rhodium(I)-Komplexe von 31 und 32 ist im Anschluß an die Beispiele für die beiden Verfahren beschrieben (vide infra). Zur weiteren Illustration der experimentellen Prozedur werden fünf dieser Hydrierungen im Detail beschrieben:

a) Hydrierung unter Einsatz des neutralen Rhodium(I)-Komplex von (+)-(2R,4R)-BPPM (Formel 21) in Gegenwart von (+)-(R)-1-Phenylethylamin.

Die Suspension von 22.7 mg (0.092 mmol Rh) Bis(1,5-cyclooctadien)-dirhodium(I)-dichlorid (Fluka) in 5 ml Methanol p.A. wird in einer Schüttelente mittels eines durchperlenden Argonstroms entgast. 55.4 mg (0.10 mmol) (+)-BPPM werden zugegeben und die entstehende Lösung noch 15 weitere Min. entgast. In einem zweiten Gefäß werden 1.03 g (5.0 mmol) (E)-Phenylitaconsäure in 5 ml Methanol p.A. gelöst, 606 mg (5.0 mmol) (+)-Phenylethylamin (Adrich) zugegeben und auch diese Lösung mit Argon gespült. Anschließend wird diese Lösung zu der Lösung in der Schüttelente gegossen. Die Schüttelente wird an ein Wasserstoff-Vorratsgefäß angeschlossen und die Argonatmosphäre mehrmals durch Wasserstoff verdrängt. Dann wird die Lösung bei Raumtemperatur (25°C) unter konstant 1 x $10^5$ Pa (1 atm) $H_2$ 16 Stunden geschüttelt. Es werden ca. 120 ml $H_2$-Gas aufgenommen (100 % d. Th.). HPLC (Laufmittel Wasser/Acetonitril 3 : 1, 1.5 g $NH_4H_2PO_4$ pro Liter Laufmittel enthaltend und mit konz. Phosphorsäure auf pH 3.5 eingestellt) zeigt quantitativen Umsatz der Phenylitaconsäure ($t_{ret}$ 7.74 Min) zur Benzylbernsteinsäure ($t_{ret}$ 6.74 Min) an. Der Wasserstoff in der Schüttelente wird durch Stickstoff verdrängt und dann unter Kühlung 15 ml 2N Salzsäure zugetropft. Der Feststoff wird abgesaugt und das Filtrat mit dreimal je 30 ml MTB-Ether extrahiert. Die vereinigten Extrakte werden mit dreimal 40 ml 2N Salzsäure gewaschen, dann getrocknet, im Vakuum eingeengt und der Rückstand im HV getrocknet. Man erhält 900 mg (Ausb. 87 % d. Th.) farblosen Feststoff. Dieses Rohprodukt hat gemäß ®CHIRALCEL OC-Analyse 95.5 % ee der (R)-Konfiguration. Der Feststoff wird in 15 ml Wasser aufgekocht, 50 mg Aktivkohle zugesetzt, 10 Min. gekocht. Es wird heiß filtriert und mit 10 ml heißem Wasser nachgewaschen. Das Filtrat wird 12 Std. auf 0°C gekühlt, der auskristallisierte Feststoff abgesaugt, mit 5 ml Eiswasser gewaschen und im HV getrocknet.

630 mg (Ausb. 61 %) farbloser Feststoff, $[\alpha]_D^{25}$ = +26.3° (c=1.5, EtOAc). Dieses umkristallisierte Produkt hat gemäß ®CHIRALCEL OC-Analyse 99.2 % ee.

Tabelle 1a: Asymmetrische Hydrierungen von Phenylitaconsäure (Formel 4) zu optisch aktiver 2-Benzylbernsteinsäure (Formel 5)

| Nr. | Bsp. | t(°C)/ Rktszeit | Diphosphin | Formel | Komplex Formel | Substrat/ Katal.-Verhältn. | Additiv (Äq. bez. auf Substrat) |
|---|---|---|---|---|---|---|---|
| 1 | - | 45°C/40h | (-)BPPM | 20 | a) | 66 | (-)$PhCH(CH_3)NH_2$ (2.0)<br>$HCO_2H$ (10.0) |
| 2 | - | 55°C/25h | (-)BPPM | 20 | a) | 80 | (-)$PhCH(CH_3)NH_3$ (2.0)<br>$HCO_2H$ (15.0) |
| 3 | - | 30°C/40h | (-)BPPM | 20 | a) | 16 | (-)$PhCH(CH_3)NH_2$ (2.0)<br>$HCO_2H$ (15.0) |
| 4 | - | 25°C/16h | (-)BPPM | 20 | 11 | 50 | $NEt_3$ (1.0) |
| 5 | - | 25°C/16h | (-)BPPM | 20 | 11 | 50 | (-)$PhCH(CH_3)NH_2$ (1.0) |
| 6 | - | 25°C/16h | (-)BPPM | 20 | 11 | 50 | (+)$PhCH(CH_3)NH_2$ (1.0) |
| 7 | 1a) | 25°C/16h | (+)BPPM | 21 | 11 | 50 | (+)$PhCH(CH_3)NH_2$ (1.0) |
| 8 | 1b) | 25°C/5h | (+)BPPM | 21 | 11 | 200 | $NEt_3$ (1.0) |

Tabelle 1a: Fortsetzung

| Nr. | Bsp. | t(°C)/ Rktszeit | Diphosphin | Formel | Komplex Formel | Substrat/ Katal.-Verhältn. | Additiv (Äq. bez. auf Substrat) |
|---|---|---|---|---|---|---|---|
| 9 | - | 25°C/16h | (+)BPPM | 21 | 11 | 1000 | $NEt_3$ (1.0) |
| 10 | - | 25°C/16h | (+)BPPM | 21 | 11 | 200 | $(C_6H_{11})_2NEt$ (1.0) |
| 11 | 1c) | 25°C/4h | (+)Phenyl-CAPP | 23 | 11 | 100 | -- |
| 12 | - | 25°C/5h | (+) | 24 | 11 | 200 | $NEt_3$ (1.0) |
| 13 | - | 25°C/48h | (-)Norphos | 25 | 11 | 200 | $NEt_3$ (1.0) |
| 14 | - | 25°C/24h | (+)-DIOP | 26 | 13 | 1000 | $NEt_3$ (1.0) |
| 15 | - | 25°C//4h | (-)Phenyl-β-glup | 27 | 11 | 200 | $NEt_3$ (1.0) |
| 16 | 1d) | 25°C/48h | (-)Phenyl-β-glup | 27 | 13 | 200 | $NEt_3$ (1.0) |

Tabelle 1a: Fortsetzung

| Nr. | Lsgsm. | $H_2$-Druck (bar) | Roh-Ausb. % nach Aufarbeitung | Roh-ee % nach Aufarbeitung | Rein-Ausb. % | Rein-ee % | Absol.-Konfig. |
|---|---|---|---|---|---|---|---|
| 17 | $CH_3OH$ | 1 | 96 | 3 | - | - | (R) |
| 18 | $CH_3OH$ | 1 | 35[d)] | 22 | - | - | (R) |
| 19 | $CH_3OH$ | 1 | 95 | 10 | 65 | 7 | (S) |
| 20 | $CH_3OH$ | 50 | 92 | 60 | 75 | 64 | (R) |
| 21 | iPrOH/$C_6H_6$ 1 : 1 | 50 | 75[e)] | 73 | 60 | 71 | (R) |
| 22 | $CH_3OH$ | 30 | 50[c)] | 32 | - | - | (R) |

a) Asymmetrische katalyt. Transferhydrierung; Einsatz von Rhodium(II)acetat-Hydrat; Prozedur analog zu H. Brunner et al., (Synthesis, 743 (1989)); b) ca. 25 % Ausgangsmaterial unumgesetzt; c) ca. 50 % Ausgangsmaterial unumgesetzt; d) ca. 65 % Ausgangsmaterial unumgesetzt; e) es entstehen Nebenprodukte

Tabelle 1b

| Asymmetrische Hydrierungen von Phenylitaconsäure (Formel 4) zu optisch aktiver 2-Benzylbernsteinsäure (Formel 5) | | | | | | | |
|---|---|---|---|---|---|---|---|
| Nr. | Lsgsm. | $H_2$-Druck (bar) | Roh-Ausb. % nach Aufarbeitung | Roh-ee % nach Aufarbeitung | Rein-Ausb. % | Rein-ee % | Absol.-Konfig. |
| 1 | DMSO | 1 | 100 | - | 77 | 31 | (R) |
| 2 | DMSO | 1 | 100 | 28 | 52 | 24 | (R) |
| 3 | DMSO | 1 | 75 | - | 40 | 15 | (S) |
| 4 | $CH_3OH$ | 1 | 90 | 94 | 60 | 98,4 | (S) |
| 5 | $CH_3OH$ | 1 | 80 | 95 | 60 | 98,5 | (S) |
| 6 | $CH_3OH$ | 1 | 80 | 96 | 55 | 99,5 | (S) |
| 7 | $CH_3OH$ | 1 | 87 | 95,5 | 61 | 99,2 | (R) |
| 8 | $CH_3OH$ | 1 | 92 | - | 75 | 97,0 | (R) |
| 9 | $CH_3OH$ | 1 | 70[b)] | - | 50 | 86,3 | (R) |
| 10 | $CH_3OH$ | 1 | 86 | - | 60 | 95,0 | (R) |
| 11 | $CH_3OH/C_6H_5$ 3 : 1 | 1 | 96 | 96 | 63 | 98,5 | (R) |
| 12 | $CH_3OH$ | 1 | 96 | - | 70 | 3 | (R) |
| 13 | $CH_3OH$ | 1 | 50[c)] | 57 | - | - | (R) |
| 14 | $CH_3OH$ | 1 | 90 | 62 | - | - | (R) |
| 15 | $CH_3OH$ | 1 | 95 | - | 75 | 4 | (R) |
| 16 | $CH_3OH$ | 1 | 75[b)] | 65 | 60 | 59 | (R) |
| 17 | $CH_3OH$ | 1 | 96 | 3 | - | - | (R) |
| 18 | $CH_3OH$ | 1 | 35[d)] | 22 | - | - | (R) |
| 19 | $CH_3OH$ | 1 | 95 | 10 | 65 | 7 | (S) |
| 20 | $CH_3OH$ | 50 | 92 | 60 | 75 | 64 | (R) |
| 21 | iPrOH/$C_6H_6$ 1 : 1 | 50 | 75[e)] | 73 | 60 | 71 | (R) |
| 22 | $CH_3OH$ | 30 | 50[c)] | 32 | - | - | (R) |

a) Asymmetrische katalyt. Transferhydrierung; Einsatz von Rhodium(II)acetat-Hydrat; Prozedur analog zu H. Brunner et al., (Synthesis, 743 (1989));
b) ca. 25 % Ausgangsmaterial unumgesetzt;
c) ca. 50 % Ausgangsmaterial unumgesetzt;
d) ca. 65% Ausgangsmaterial unumgesetzt;
e) es entstehen Nebenprodukte

b) Hydrierung unter Einsatz des neutralen Rhodium(I)-Komplexes von (+)-(2S,4S)-BPPM (Formel 21) in Gegenwart von Triethylamin.

Die entgaste Lösung von 100 g (0.48 Mol) (E)-Phenylitaconsäure in 1.0 l Methanol und 67 ml (0.48 Mol) Triethylamin wird zu der mit Argon gespülten, in einer Schüttelente befindlichen Lösung von 0.54 g (1.1 mmol) Bis(1,5-cyclooctadien)dirhodium(I)dichlorid und 1.33 g (2.4 mmol) (+)-BPPM in 460 ml Methanol gegeben. Die entstehende Lösung wird bei Raumtemperatur unter 1 atm Wasserstoffgas geschüttelt. Innerhalb von 5 Std. werden 10 l $H_2$ aufgenommen und HPLC (s.o.) zeigt quantitative Hydrierung an. Unter Eiskühlung (IT < 30°C) werden 1.5 l 2N Salzsäure zugetropft. Der Niederschlag wird abgesaugt und das Filtrat im Vak. auf ca. 1.5 l aufkonzentriert. Man extrahiert einmal mit 2 l und zweimal mit 1 l MTB-Ether und wäscht die vereinigten Extrakte mit viermal 1 l 2N Sälzsäure. Man trocknet die Etherphase, engt im Vakuum zur Trockne ein (93 g Feststoff), nimmt in 1.5 l Wasser auf, erhitzt zum Sieden, setzt 3 g Aktivkohle zu und kocht 10 Min. Man filtriert heiß und wäscht mit zweimal je 100

ml kochendem Wasser nach. Das Filtrat wird 3 Tage bei 0°C aufbewahrt, der auskristallisierte Feststoff abgesaugt, mit 500 ml Eiswasser gewaschen und im HV über $P_2O_5$ getrocknet.

Man erhält 74.6 g (0.36 Mol, Ausb. 75 % d.Th.) farblosen Feststoff, $[\alpha]_D^{25}$ = +25.7° (c=1.5, EtOAc), gemäß ®CHIRALCEL OC-Analyse 97 % ee der (R)-Konfiguration.

c) Hydrierung unter Einsatz des neutralen Rhodium(I)-Komplexes von (+)-(2R,4R)-Phenyl-CAPP (Formel 23) ohne Basenzusatz.

Die entgaste Lösung von 5.12 g (25.0 mmol) (E)-Phenylitaconsäure in 22 ml Methanol abs. und 8 ml Benzol wird zu der mit Argon gespülten Lösung von 62 mg (0.125 mmol) Bis(1,5-cyclo-octadien)dirhodium(I)dichlorid und 155 mg (0.27 mmol) (+)-Phenyl-CAPP in 15 ml Methanol abs. und 5 ml Benzol gegeben. Die Lösung wird bei Raumtemperatur unter 1 x $10^5$ Pa (1 atm) $H_2$ geschüttelt. Die $H_2$-Aufnahme setzt nach wenigen Minuten ein. Innerhalb von 4 Std. werden 550 ml $H_2$-Gas (100 % d. Th.) aufgenommen und HPLC (s.o) zeigt quantitative Hydrierung an. Die Lösung wird mit Stickstoff gespült, dann 40 g Eis und 40 ml 2N Salzsäure zugesetzt. Ein gelblicher Feststoff fällt in größerer Menge aus. 100 ml MTB-Ether werden zugesetzt und gründlich gerührt. Die geringe Menge verbleibender Feststoff wird abgesaugt und mit MTB-Ether gewaschen. Die organische Phase des Filtrats wird abgetrennt und die wäßrige Phase noch mit dreimal je 100 ml MTB-Ether extrahiert. Die vereinigten Ether-Phasen werden mit zweimal 100 ml 1N Salzsäure gewaschen, dann getrocknet und das Lösungsmittel im Vak. entfernt. Der Rückstand wird im HV getrocknet. Man erhält 5.0 g (24.0 mmol, Ausb. 96 % d. Th.) fast weißen Feststoff. Dieses Rohprodukt hat gemäß ®CHIRALCEL OC-Analyse 96 % ee der (R)-Konfiguration.

Das Rohprodukt wird in 75 ml Wasser aufgenommen und in der Siedehitze 300 mg Aktivkohle zugesetzt. Man kocht 10 Min., saugt heiß ab und wäscht mit 10 ml heißem Wasser nach. Das Filtrat wird 2 Tage bei 0°C gehalten. Die Kristalle werden abgesaugt, mit 25 ml Eiswasser gewaschen und im HV über $P_2C_5$ getrocknet. Man erhält 3.26 g (15.7 mmol, 63 % d. Th.) farblosen Feststoff, Schmp. 164-165°C, $[\alpha]_D^{25}$ = +26.9° (c=1.45, EtOAc). Dieser Feststoff hat gemäß ®CHIRALCEL-OC-Analyse 98.5 % ee und (R)-Konfiguration.

d) Hydrierung unter Einsatz des kationischen Rhodium(I)-Komplex von Phenyl-β-glup (Formel 27) ohne Basenzusatz.

Die Argon-gespülte Lösung von 39 mg (0.055 mmol) Phenyl-β-glup (ISIS-Chemie) in 50 ml Methanol p.A. wurde in eine Schüttelente gegeben, die 20 mg (0.05 mmol) Bis(1,5-cyclooctadien)-rhodium(I)-tetrafluoroborat (Aldrich) unter Argon-Atmosphäre enthielt. Die Argon-gespülte Lösung von 2.06 g (10.0 mmol) (E)-Phenylitaconsäure in 100 Methanol wurde zugefügt und die entstehende Lösung unter 1 x $10^5$ Pa (1 atm) Wasserstoff geschüttelt. Eine langsame $H_2$-Aufnahme flaute nach 48 Std. vollständig ab. Zu diesem Zeitpunkt waren 215 ml aufgenommen worden. HPLC zeigte 75 % Produkt und 25 % Ausgangsmaterial an. Die Lösung wurde mit $N_2$ gespüft, 60 ml MTB-Ether und 32 ml 1N Salzsäure zugesetzt und 15 Min. gerührt. Der Feststoff wurde abgesaugt. Die organische Phase des Filtrats wurde abgetrennt und die wäßrige Phase mit 40 ml MTB-Ether extrahiert. Die vereinigten organischen Phasen wurden mit zweimal 30 ml 0.5 N Salzsäure gewaschen, dann getrocknet, das Lösungsmittel im Vakuum entfernt und der Rückstand im HV getrocknet. Man erhält 2.1 g (100 % d. Th.) eines Gemischs von Öl und Feststoff, $[\alpha]_D^{22}$ = +10.2° (c=1.44, EtOAc). Unter Berücksichtung des ca. 25 %igen Gehalts an (E)-Phenylitaconsäure berechnet sich daraus ein ee der (R)-Benzyl-bernsteinsäure von ca. 55 %. ®CHIRALCEL OC-Analyse, bei der beide Enantiomere des Produkts gut vom Ausgangsmaterial getrennt werden, ergab 65 % ee. Nach Umkristallisation aus heißem Wasser/Aktivkohle (wie bei c)) war der ee auf 58.6 % gesunken.

e) Hydrierung unter Einsatz des kationischen Ruthenium(II)-Komplexes (Formel 16) von (S)(-)-BINAP (Formel 33)

Die Herstellung des Ruthenium(II)-Komplexes folgte der Kurzmitteilung von K. Mashima et al. J. Chem. Soc., Chem. Commun., 1208 (1989) und ist in der vorliegenden Anmeldung detailliert beschrieben (vide infra).

Zu 50 ml argondurchperltem Methanol wurden 5.15 g (25 mmol) (E)-Phenylitaconsäure und 28 mg (0,025 mg) des Komplexes RuI[((S)-BINAP)(p-$CH_3$-$C_6H_4$-$CH(CH_3)_2$)]$^+I^-$ gegeben. Dann wurde in einem Autoklaven 1 Tag bei 20°C unter 30 bar $H_2$ geschüttelt. HPLC zeigte 52 % Ausgangsmaterial und 48 % Produkt an. Das Lösungsmittel wurde im Vak. entfernt, der Rückstand in 150 ml MTB-Ether aufgenommen und 5 ml 1N Salzsäure zugesetzt. Der Niederschlag wurde abgesaugt. Im Filtrat wurde die MTB-Ether-Phase abgetrennt, mit 10 ml Wasser gewaschen, dann getrocknet und das Lösungsmittel im Vak. entfernt.

Ausbeute: 5.2 g (100 % d. Th.). Gemäß ®CHIRALCEL OC-Analyse hat der hydrierte Produktanteil 32 % ee der (R)-Konfiguration.

$[\alpha]_D^{20}$ = + 3.9° (c = 1.45, EtOAc). Die daraus berechnete optische Reinheit von 14.3 % ee führt wegen des Gehalts von 52 % unhydrierter Phenylitaconsäure ebenfalls zu ca. 30 % ee des hydrierten Produktanteils.

Stufe 3:

Optisch aktiver (R)-Benzylbernsteinsäure-bis(p-nitrophenylester), Formel 6, ($R^3 = R^4 = H$, $R^5$ = p-Nitrophenyl)

Zur Suspension von 78.9 g (0.378 Mol) (R)-Benzylbernsteinsäure (97 % ee) in 800 ml Toluol gibt man auf einmal 158 g (0.757 Mol, 2.0 Äquiv.) Phosphorpentachlorid und erwärmt unter $N_2$ gelinde auf 40°C. Es tritt in einer anfangs leicht exothermen Reaktion eine heftige HCl-Gasentwicklung ein. Man rührt eine Std. bei 40 bis max. 45°C. Das Toluol wird im Vakuum bei 35°C Badtemperatur weitgehend abgezogen. Das zurückbleibende gelbe Öl (110 g, Theorie 92 g, d. h. 18 g Resttoluol) wird unter Feuchtigkeitsausschluß in 400 ml Dichlormethan gelöst und 111 g (0.794 Mol, 2.09 Äquiv.) p-Nitrophenol werden zugegeben. Die grünliche Suspension wird auf 15°C abgekühlt und innerhalb 40 Min. 97 g (0.95 Mol, 2.5 Äquiv.) Triethylamin zugetropft. Nach Zugabe von 1/3 bis 1/2 des Amins entsteht eine klare Lösung, die beim weiteren Zutropfen wieder in eine Suspension übergeht. Die Innentemperatur wird während des gesamten Zutropfens bei 15-20°C gehalten. Danach rührt man 20 Std. bei Raumtemperatur. Man verdünnt mit 500 ml Dichlormethan und verrührt anschließend jeweils 5 Min viermal mit je 500 ml halbkonzentrierter Natriumcarbonat-Lösung. Die wäßrigen Phasen werden jeweils abgetrennt. Die vereinigten wäßrigen Phasen werden filtriert und nochmals mit 500 ml Dichlormethan extrahiert. Die vereinigten Methylenchloridphasen werden mit 500 ml Wasser gewaschen, dann getrocknet und das Lösungsmittel im Vakuum entfernt. Man erhält 156 g (0.346 Mol, 92 % d. Th.) eines grünlich-braunen Feststoffs als Rohprodukt. Dieses wird in 500 ml Dichlormethan klar gelöst, auf 0°C abgekühlt und 3.5 l Diisopropylether innerhalb 30 Min. unter Rühren zugetropft, wobei nach ca. 600 ml ein dicker, nur schwer rührbarer Brei entsteht, der bei weiterer Zugabe in eine gut rührbare Suspension übergeht. Der Feststoff wird kalt abgesaugt, mit 500 ml kaltem Diisopropylether gewaschen, trockengesaugt und im HV getrocknet. Man erhält 137 g (0.304 Mol, 80 % d. Th.) eines hellgrauen Feststoffs, Schmp. 114 - 118°C (Zers.).

$^1$H-NMR ($CDCl_3$): $\delta$ = 8.2-8.3 (m,4H,arom-H), 7.2-7.45 (m,7H,arom-H), 7.10 (m,2H,arom-H), 3.50 (m,1H,$C\underline{H}CH_2Ph$), 3.25 (dd,1H), 3.0-3.2 (m,2H), 2.88 (dd,1H) ppm.
MS (DCl, Isobutan): m/e = 451 (M + $H^+$), 312 ($M^+$-Nitrophenol), 173 ($M^+$- 2-Nitrophenol-H).

Ausgehend von racemischer Benzylbernsteinsäure wurde ein Dinitrophenylester niedrigeren Schmelzpunkts (104-106°C) erhalten.

Stufe 4:

Optisch aktives 2(R)-Benzylbernsteinsäure-1-(p-nitrophenyl-ester)-4-(4-Boc-amino-1-piperidid), (Formel 1, $R_1R_2N$ = 4-Boc-Aminopiperidyl, $R^3 = R^4 = H$, $R^5$ = p-Nitrophenyl).

Zur Lösung von 135 g (0.30 Mol) des (R)-Diesters (Stufe 3) in 1.0 l N,N-Dimethyfformamid gibt man bei -10°C unter $N_2$ innerhalb 10 Min. 60 g (0.30 Mol) 4-Boc-Aminopiperidin. Man rührt 45 Min. bei 0°C. DC (Kieselgel, $CH_2Cl_2$/EtOAc 5 : 1) zeigt vollständige Umwandlung des Diesters ($R_f = 0.87$) in das Produkt ($R_f = 0.35$) und p-Nitrophenol ($R_f = 0.59$) an.
Man gibt 6.5 l Wasser und 1.2 l Dichlormethan zu, rührt durch und trennt die organische Phase ab. Die wäßrige Phase wird nochmals mit zweimal 1.0 l Dichlormethan extrahiert. Die vereinigten organischen Phasen werden mit viermal 250 ml 0.5 N Natronlauge gewaschen, um das Nitrophenol zu entfernen.
Anschließend wird mit 250 ml Wasser gewaschen. Die Methylenchlorid-Phase wird getrocknet und das Lösungsmittel im Vakuum entfernt. Man erhält als Rohprodukt 144.3 g (0.282 Mol, 94 % d. Th.) eines hellbraunen Schaums. Kristallisation erfolgt aus Acetonitril. Man erhält 94.2 g (0.184 Mol, 62 % d. Th.) farblosen Feststoff, Schmp. 103 - 105°C, $[\alpha]_D^{25}$ = +23.9° (c=1.54, EtOAc).

$^1$H-NMR ($CDCl_3$): $\delta$ = 8.20 (dd,2H,arom-H), 7.2-7.4 (m,5H,arom-H), 7.08 m,2H,arom-H), 4.4-4.5 (m,2H,CO-N-$C\underline{H_2}$), 3.6-3.8 (m,2H,CO-N-$C\underline{H_2}$), 3.40 (m,1H,NH-$C\underline{H}$), 3.10 (m,2H,N-CO-$C\underline{H_2}$), 2.68-3.00 (m,3H,$N\underline{H}$ und $PhC\underline{H_2}$), 2.58 (dt,1H,$PhCH_2C\underline{H}$), 1.9-2.1 (br.t,2H,NH-CH-$C\underline{H_2}$), 1.44 (s,9H,tert.-Bu), 1.20-1.47 (m,2H,NH-CH-$C\underline{H_2}$). MS (DCl, Isobutan): m/e = 512 (M + $H^+$), 456, 373.

Beispiel 2

Racemisierungsfreie Umsetzung des Aktivesters der Formel 1 (Verfahren 1, Stufe 4) mit aliphatischen Aminen.
2.56 g (5.0 mmol) des Aktivesters (Verfahren 1, Stufe 4) werden in 25 ml DMF klar gelöst. 770 mg (6.35 mmol) (S)-(-)-1-Phenylethylamin werden zugesetzt und das Gemisch dann 3 Std. auf 40-45°C erwärmt. DC ($CH_2Cl_2$/EtOAc 5 : 1) zeigt vollständige Umsetzung des Aktivesters ($R_f = 0.26$) zum Produkt ($R_f = 0.1$) und p-Nitrophenol ($R_f = 0.64$) an und nur geringe Reste des Amins ($R_f = 0.04$). Man kühlt auf 0°C, setzt 150 ml Wasser zu und extrahiert mit 2 x 50 ml Dichlormethan. Die Extrakte werden getrocknet und das Lösungsmittel im HV entfernt. Man erhält 2.3 g (4.66 mmol, 93 % d. Th.) des Rohprodukts (Öl-Feststoff-Gemisch). HPLC des Rohprodukts über MOS Hypersil unter den im Kapitel "Allgemeine Arbeitstechnik und Analytik" angegebenen Bedingungen , zeigt nur das (RS)-Diastereomer der Formel 34 ($t_{ret}$ 8.05 Min.), nicht das (SS)-Diastereomer von 34 ($t_{ret}$ 7.58 Min) an. Geht man von racemischem Aktivester der Formel 1 aus, so erhält man die beiden Diastereomeren von 34 im Verhältnis 1 : 1.

Zur Entfernung des p-Nitrophenols aus dem Rohprodukt löst man das Rohprodukt unter gelindem Erwärmen in 100 ml Acetonitril, kühlt auf ca. 10°C, setzt 200 ml Wasser zu und rührt 20 Min im Eisbad. Man saugt den Feststoff ab und trocknet ihn im Vakuum: 1.5 g weiße Kristalle; reines (RS)-Diastereomer der Formel 34. Unter entsprechenden Reaktionsbedingungen ergibt der Aktivester auch mit solchen Aminen, die C-terminale Teile von Reninhemmern sind (z. B. solche aus DE-A 39 30 397), racemisierungsfreie Amidbildung.

Beispiel 3

Verseifung des Aktivesters der Formel 1

Die Lösung von 2.56 g (5.0 mmol) des Aktivesters (Beispiel 1, Stufe 4) in 150 ml Acetonitril wird unter $N_2$ mit 50 ml 0.1 N Natronlauge (5.0 mmol) versetzt und 3 Std. auf 40-45°C erwärmt. Da die Reaktion gemäß DC unvollständig bleibt, wird 80 mg (2.0 mmol) Natriumhydroxid zugesetzt und noch 15 Min. bei 40-45°C gerührt. Nun zeigt DC nur noch Nitrophenol und Carbonsäure der Formel 1 an. Man entfernt das organische Lösungsmittel bei 30°C im Vakuum. Der wäßrige Rückstand wird mit 2 N Salzsäure auf pH 1 gestellt. Der ausfallende Feststoff wird mit 2 x 50 ml Dichlormethan extrahiert. Die Extrakte werden getrocknet, das Lösungsmittel im Vak. entfernt. Das Rohprodukt zeigt keine optische Drehung $[\alpha]_D^{20}$ = 0° (c=1.0, Methanol) und ist gemäß ®CHIRALCEL OC-Analyse racemisch.

Beispiel 4

Beispiele zum erfindungsgemäßen Verfahren

Stufe 1:

identisch mit Beispiel 1, Stufe 1.

Stufe 2:

Phenylitaconsäureanhydrid (Formel 7, $R^3 = R^4 = H$)

Zur Lösung von 2.28 kg (11.1 Mol) (E)-Phenylitaconsäure in 5.7 l THF gibt man bei 20°C 2.3 l (ca. 2.49 kg, ca. 24.4 Mol) Essigsäureanhydrid. Man rührt 5 Std. bei Raumtemperatur. Nach 1 Std. liegt eine klare hellgelbe Lösung vor, nach 3 Std. beginnt das Produkt auszukristallisieren. Man entfernt das THF im Vak., fügt dem Rückstand zur Vervollständigung der Kristallisation 5.0 l Methyl-tert.-butylether zu und saugt das Produkt ab. Es wird mit MTB-Ether gewaschen, trockengesaugt und im Vak. bei 40°C getrocknet. Ausbeute: 1.68 kg (8.94 Mol, 81 % d. Th.), Schmp. 167-170°C.

$^1$H-NMR (DMSO-$d_6$): $\delta$ = 7.63-7.73 (m,3H,=CH + 2 arom.-H), 7.47-7.56 (m,3H, 3 arom.-H), 3.99 (d,2H,$CH_2$) ppm.

Stufe 3:

2-(E)-Benzyliden-bernsteinsäure-4[(4-Boc-amino)-1-piperidid] (Formel 8, $R^1R^2N$ = 4-Boc-aminopiperidyl, $R^3 = R^4 = H$)

Zur Suspension von 2.29 kg (12.2 Mol) Phenylitaconsäureanhydrid in 20 l Ethylacetat gibt man bei 24°C in gleichmäßigen Portionen innerhalb von 90 Min. 2.81 kg (14.05 Mol, 1.15 Äquiv.) 4-(Boc-Amino)-piperidin. Man rührt 4 Std. bei 25°C nach, wobei ein dicker weißer Niederschlag entsteht. Man läßt 20 l MTB-Ether innerhalb 30 Min zulaufen und rührt die Suspension 2 Std. bei 10°C. Der Feststoff wird abgesaugt und mit 2mal 2 l MTB-Ether gewaschen. Er wird trockengesaugt und dann bei 40°C im Vak. getrocknet. Ausbeute: 3.76 kg (9.7 Mol, 80 % d. Th.) farbloser Feststoff, Schmp. 157-160°C.

$^1$H-NMR (DMSO-$d_6$): $\delta$ = 12.47 (s,1H,$CO_2H$), 7.72 (s,1H, =C$\underline{H}$Ph), 7.30 - 7.50 (m,5H,arom.-H), 6.92 (d,1H,N$\underline{H}$Boc), 4.23 (d,1H,CO-NC$\underline{H}_2$), 3.88 (d,1H,CO-NC$\underline{H}_2$), 3.50 (br,m,1H,NH-C$\underline{H}$), 3.45 (s,2H,N-CO-C$\underline{H}_2$-C=), 3.10 (t,1H,CO-NC$\underline{H}_2$), 2.74 (t,1H,CO-NC$\underline{H}_2$), 1.76 (m,2H,NH-CH-C$\underline{H}_2$), 1.39 [s,9H,C(C$\underline{H}_3$)$_3$], 1.14 - 1.38 (m,2H,NH-CH-C$\underline{H}_2$) ppm.

Bestimmung des Regioisomeren-Verhältnisses durch symmetrische Hydrierung/HPLC-Analyse ergab > 90 % des 4-Amids.

Stufe 4:

Optisch aktives 2-(R)-Benzylbernsteinsäure-4-[4(-Boc-amino)-1-pipendid], Formel 1, $R^1R^2N$ = 4-Boc-Amino-piperidyl, $R^3 = R^4$ = H, $R^5$ = H)

Einen Gesamtüberblick über die durchgeführten asymmetrischen Hydrierungen von Stufe 3, die dabei erzielten optischen Induktionen und die zugehörigen Reaktionsparameter gibt Tab. 2. Die Herstellung der neuen chiralen Diphosphine 23, 30, 31, 32 sowie der isolierten kationischen Rhodium-(I)-Komplexe von 31 und 32 ist im Anschluß an die Beispiele für das erfindungsgemäße Verfahren beschrieben (vide infra). Zur weiteren Illustration der experimentellen Prozedur werden drei dieser Hydrierungen im Detail beschrieben:

a) Hydrierung unter Einsatz des kationischen Rhodium(I)-Komplexes von (3R,4R)(+)-1-Phenylaminocarbonyl-3,4-bis-(diphenylphosphino)pyrrolidin (Formel 1) bei 50 x $10^5$ Pa (50 bar) $H_2$-Druck

Zur Argon-durchperlten Lösung von 840 g (2.16 Mol) der Stufe 3 in 11 l Methanol p.A. gibt man 18.5 g (21.6 mmol, Substrat/Katalysator-Verhältnis 100 : 1) [(3R,4R)-1-Phenylaminocarbonyl-3,4-bis(diphenylphosphino)pyrrolidin-P,P'](1,5-cyclooctadien)-rhodium-tetrafluoroborat. Man hydriert 24 Std. bei 20°C unter 50 x $10^5$ Pa (50 bar) $H_2$-Druck. HPLC-Analyse zeigt quantitative Reaktion an. Das Lösungsmittel wird bei 30°C Badtemperatur im Vak. vollständig entfernt. Der Rückstand wird in 20 l MTB-Ether aufgenommen und mit 8 l 0.5 N Salzsäure ausgerührt. Die Wasserphase wird mit 2mal 5 l MTB-Ether extrahiert. Die vereinigten Ether-Phasen werden mit 2mal 2 l 0.5 N Salzsäure gewaschen und dann getrocknet. Das Lösungsmittel wird im Vak. entfernt und der Rückstand im HV zur Gewichtskonstanz getrocknet. Das Rohprodukt (800 g) zeigt bei Analyse auf ®CHIRALCEL OC etwa 80 % ee der (R)-Konfiguration.

Das Rohprodukt wird in 10 l Diethylether suspendiert und mit Methanol/Diisopropylether kristallisiert. Ausbeute: 687 g (1.76 Mol, 81.3 % d.Th.) farbloser Feststoff, Schmp. 135 - 136°C. ®CHIRALCEL OC-Analyse ergibt 93 % ee der (R)-Konfiguration.

$^1$H-NMR (DMSO-$d_6$): $\delta$ = 12.05 (s,1H,$CO_2H$), 7.32-7.15 (m,5H,arom.-H), 6.75-6.90 (dd,br.,1H,NH), 4.15 (d,br.,1H,NCH), 3.71 (d,br.,1H,NCH), 3.43 (s,br.,1H,NCH), 2.53-3.05 (m,6H,CO-C$\underline{H}_2$,Ph-C$\underline{H}_2$,NCH), 2.27 (dd,1H,C$\underline{H}CO_2H$), 1.68 (t,br,2H,NCHC$\underline{H}_2$), 1.36 (s,9H,$C(CH_3)_3$), 1.05-1.25 (m,2H,NCHC$\underline{H}_2$) ppm.

Tabelle 2a

| Asymmetrische Hydrierungen von 2-(E)-Benzyliden-bernsteinsäure-[(4-Boc-amino)-1-piperidid] (Formel 8) zu optisch aktivem 2-(R)-Benzylbernsteinsäure-4-[(Boc-amino)-1-piperidid] (Formel 1) | | | | | | | |
|---|---|---|---|---|---|---|---|
| Nr. | Bsp. | t(°C)/Rktszeit | Diphosphin | Formel | Komplex Formel | Substrat/Katal.-Verhältn. | Additiv (Äq. bez. auf Substrat) |
| 1 | 4a) | 20°C/24h | (+) | 31 | 13 | 100 | -- |
| 2 | 4a$_1$) | 25°C/24h | (+) | 31 | 13 | 100 | -- |
| 3 | 4a$_2$) | 25°C/24h | (+) | 32 | 13 | 100 | -- |
| 4 | 4b) | 20°C/24h | (+) | 31 | 13 | 300 | -- |
| 5 | - | 25°C/24h | (+) | 31 | 13 | 200 | -- |
| 6 | - | 25°C/24h | (+) | 30 | 13 | 100 | -- |
| 7 | 4c) | 25°C/2h | (+)-Phenyl-CAPP | 23 | 11 | 100 | -- |
| 8 | - | 25°C/16h | (-)-BDPP | 29 | 13 | 200 | -- |
| 9 | - | 25°C/18h | (-)-Phenyl-β-glup | 27 | 13 | 200 | -- |
| 10 | - | 25°C/24h | (-)BPPM | 20 | 11 | 230 | $NEt_3$ (0,96) |

a) Es entstehen ca. 10 %

HPLC-sichtbare Nebenprodukte

Tabelle 2b

| Asymmetrische Hydrierungen von 2-(E)-Benzyliden-bernsteinsäure-[(4-Boc-amino)-1-piperidid] (Formel 8) zu optisch aktivem 2-(R)-Benzylbernsteinsäure-4-[(Boc-amino)-1-piperidid] (Formel 1) | | | | | | | |
|---|---|---|---|---|---|---|---|
| Nr. | Lsgsm. | $H_2$-Druck (bar) | Roh-Ausb. % nach Aufarbeitung | Roh-ee % nach Aufarbeitung | Rein-Ausb. % | Rein-ee % | Absol.-Konfig. |
| 1 | $CH_3OH$ | 50 | 95 | 80,4 | 81 | 93,0 | (R) |
| 2 | $CH_3OH$ | 50 | 95 | 72,4 | 71 | 95,4 | (R) |
| 3 | $CH_3OH$ | 50 | 95 | 81 | -- | -- | (R) |
| 4 | $CH_3OH$ | 100 | 96 | 77 | 64 | 94,6 | (R) |
| 5 | $CH_3OH$ | 100 | 100 | -- | 70 | 98,1 | (R) |
| 6 | $CH_3OH$ | 50 | 98 | 67 | 75 | 67 | (R) |
| 7 | $CH_3OH/C_6H_6$ 2,5 : 1 | 1 | 92 | 85 | 72 | 94,0 | (R) |
| 8 | $CH_3OH$ | 1 | 87[a)] | 31 | 60 | 29 | (R) |
| 8 | $CH_3OH$ | 1 | 95 | 0 | -- | -- | racem. |
| 10 | $CH_3OH$ | 1 | 95 | 18 | -- | -- | (S) |

a) Es entstehen ca. 10 % HPLC-sichtbare Nebenprodukte

$a_1$) Ein analoger Ansatz (50 bar $H_2$, Substrat/Katalysator-Verhältnis 100 : 1) mit 7.76 g (20 mmol) Substrat ergab ein Rohprodukt mit 72.4 % ee und ein umkristallisiertes Produkt mit 70.5 % Ausbeute und 95.4 % ee der (R)-Konfiguration.

$a_2$) Ein analoger Ansatz (50 x $10^5$ Pa (50 bar) $H_2$, Substrat/Katalysator-Verhältnis 100:1) mit 3.88 g (10 mmol) Substrat unter Verwendung des Katalysators [3R,4R]-1-Phenylaminocarbonyl-3,4-bis-(di-p-tolylphosphino)pyrrolidin-P,P'](1,5-cyclooctadien)-rhodium-tetrafluoroborat ergab ein Rohprodukt mit 81 % ee der (R)-Konfiguration.

b) Hydrierung unter 100 x $10^5$ Pa (100 bar) $H_2$-Druck bei höherem Substrat/Katalysator-Verhältnis.

Zur Argon-durchperlten Lösung von 7.8 g (20.1 mmol) der Stufe 3 in 100 ml Methanol wurden 58 mg (0.068 mmol, Substrat/Katalysator-Verhältnis 300 : 1) des Komplexes wie bei a) gegeben. Dann wurde 24 Std. bei 20°C unter 100 x $10^5$ Pa (100 bar) $H_2$ in einem Schüttelautoklaven hydriert. Aufarbeitung analog zu a) ergab 7.55 g (96 % d. Th.) Rohprodukt mit 77 % ee der (R)-Konfiguration.

Umlösen in Analogie zu a) ergab 5.0 g (64 % d.Th.) farblose Kristalle, die 94.6 % ee aufwiesen.

c) Hydrierung bei 1 bar $H_2$ unter Verwendung des neutralen Rhodium(I)-Komplexes von (2R,4R)-(+)-Phenyl-CAPP (Formel 23) in Methanol/Benzol.

Zur Argon-durchperlten Suspension von 31 mg (0.125 mmol Rh) Bis(1,5-cyclootadien)dirhodium(I)-dichlorid in 25 ml Methanol und 10 ml Benzol gibt man 77.5 mg (0.135 mmol) (2R,4R)-Phenyl-CAPP. Man rührt die entstehende klare Lösung 15 Min unter Argon und gibt dann 4,85 g (12.5 mmol, Substrat/Katalysator-Verhältnis 100) der Stufe 3 zu. Die Argon-Atmosphäre wird durch Wasserstoff verdrängt und die Lösung dann bei 24°C unter 1 x $10^5$ Pa (1 bar) $H_2$ geschüttelt. Innerhalb von 2 Std. wird die theoretische Menge (280 ml) $H_2$-Gas aufgenommen und HPLC zeigt quantitative Reaktion an. Die Lösungsmittel werden im Vak. entfernt. Der Rückstand wird in 100 ml MTB-Ether aufgenommen, mit 40 ml 0.5 N Salzsäure geschüttelt und Ungelöstes abgesaugt. Die wäßrige Phase des Fiftrats wird nochmals mit 50 ml MTB-Ether extrahiert. Die vereinigten Ether-Phasen werden mit 2mal 20 ml 0.5 N Salzsäure gewaschen, dann getrocknet. Das Lösungsmittel wird im Vakuum entfernt und der Rückstand im HV getrocknet. Man erhält als Rohprodukt 4.5 g (11.5 mmol, 92 % d.Th.) weißen Schaum, der gemäß ®CHIRALCEL OC-Analyse 85 % ee der (R)-Konfiguration hat.

Der Feststoff wird in 50 ml Diethylether gelöst, wobei sich sofort wieder ein weißer Niederschlag bildet. 10 ml Disopropylether werden zugegeben, 1 Std. bei -15°C gehalten, der Feststoff abgesaugt und mit 2mal 10 ml kaltem Ether gewaschen. Man trocknet im HV und erhält 3.50 g (9.0 mmol, 72 % d.Th.) farbloses Pulver, Schmp. 134 - 135°C, 94 % ee der (R)-Konfiguration. Aus der Mutterlauge fallen beim Stehen weitere 350 mg aus. Gesamtausbeute: 3.85 g (9.9 mmol, 79 % d.Th.).

Beispiel 5

Herstellung neuer chiraler Diphosphine, sowie neuer Rhodium-Katalysatoren

a) (2R,4R)(+)-Phenyl-CAPP (Formel 23)

$a_1$) (2R)-Diphenylphosphinomethyl-(4R)-diphenylphosphinopyrrolidin

Zu 20 ml Argon-gespülter frisch destillierter Trifluoressigsäure werden bei 0°C 4.04 g (7.3 mmol) (2R,4R)(+)-BPPM (Formel 21, G.L. Baker, J.K. Stille et al., J. Org. Chem. 46, 2954 (1981)) gegeben und dann 1 Std. bei 0°C gerührt. Die Trifluoressigsäure wird bei 20°C im Vak. entfernt, dann mit Argon belüftet. Der ölige Rückstand wird in 20 ml entgastem Dichlormethan gelöst und 16 ml einer mit Argon gespülten 10 %igen wäßrigen Ammoniaklösung zugegeben. Das Zweiphasengemisch wird in einem Scheidetrichter unter Argon geschüttelt, die organische Phase abgetrennt, mit Argon gespült und mit zweimal je 10 ml entgastem Wasser gewaschen. Die organische Phase wird unter Argon über 1 g Magnesiumsulfat getrocknet, das Lösungsmittel im Vak. entfernt und der Rückstand im HV getrocknet. Man erhält 2.8 g (6.15 mmol, 84 % d. Th.) eines Harzes, $[\alpha]_D^{20}$ = +13.2° (c = 1.0, Benzol), aus dem man durch Umkristallisieren aus n-Hexan unter Argon unter erheblichen Verlusten einen farblosen Feststoff, Schmp. 103-104°C, $[\alpha]_D^{20}$ = +15.75° (c = 1.0, Benzol) erhält.

$^1$H-NMR ($CDCl_3$): $\delta$ = 7.2-7.5 (m,2OH,arom.-H), 2.9-3.2 (m,3H,$NHCH_2$ und $NHC\underline{H}CH_2$), 2.80 (m,1H,$Ph_2PC\underline{H}$), 2.42 (dd.1H,$Ph_2PC\underline{H_2}$), 2.05-2.35 (m,3H,$N\underline{H}$ und $Ph_2PC\underline{H_2}$ und $C\underline{H_2}CHPPh_2$), 1.38 (m,1H,$C\underline{H_2}CHPPh_2$) ppm.

$a_2$) 1-Phenylaminocarbonyl-(2R)-diphenylaminomethyl-(4R)-diphenylphosphinopyrrolidin [(2R,4R)(+)-Phenyl-CAPP]

Die mit Argon entgasten, auf 0°C gekühlten Lösungen von 2.6 g (5.7 mmol) des Rohprodukts aus $a_1$) in 30 ml Methylenchlorid und von 0.72 g (6.0 mmol) Phenylisocyanat in 30 ml Methylenchlorid werden vereinigt und 15 Min. im Eisbad unter Argon gerührt. Das Lösungsmittel wird im Vak. entfernt und der Rückstand mit dem Laufmittel Dichlormethan/0.1 % Triethylamin unter $N_2$-Druck über 30 g Kieselgel (35-70 $\mu$) chromatographiert. Man erhält 2.18 g (3.8 mmol, 67 % d. Th.) farblose Kristalle, Schmp. 180-182°C, $[\alpha]_D^{25}$ = +21.9° (c=0.5, Benzol).

$^1$H-NMR ($CDCl_3$): $\delta$ = 7.2-7.6 (m,24H,arom.-H), 6.98 (m,1H,arom.-H), 5.80 (s,1H,NH), 4.08 (m,1H,NCH), 3.86 (t,1H,$NCH_2$), 3.30 (dd,1H,$NCH_2$), [2.99 (dt,1H), 2.80-2.95 (m,1H), 2.30-2.42 (m,1H), 2,28 (dd,1H) , $C\underline{H_2}PPh_2$ und $C\underline{H_2}CHPPh_2$], 1.98 m, (angenähert qui,1H, $C\underline{H}PPh_2$] ppm.

b) (3R,4R)(+)-1-Acetyl-3,4-bis(di-p-tolylphosphino)-pyrrolidin (Formel 30)

$b_1$) Di-(p-tolyl)-phospan wird gemäß J.Am.Chem.Soc.95, 210 (1973) durch Reduktion des entsprechenden Chlorphosphans mit Lithiumaluminiumhydrid in refluxierendem Diethylether hergestellt und im HV destilliert: Sdp. 135-137°C/1 Torr, Ausbeute 71 % d. Th.

$b_2$) (3S,4S)-3,4-Bis(methylsulfonyloxy)-pyrrolidinium-bromid wird gemäß Deutsche Offenlegungsschrift DE 3403194 (Offenlegungstag 1.8.1985) aus L-(+)-Weinsäure hergestellt.

$b_3$) (3R,4R)-3,4-Bis(di-p-tolylphosphino)-pyrrolidinium-chlorid

Zur Lösung von 33.15 g (155 mmol) Di-(p-tolyl)-phosphan in 150 ml abs. THF werden 3.56 g (155 mmol) Natrium gegeben und 5 Std. unter $N_2$ unter Rückfluß gekocht, wobei das Natrium weitgehend unter Reaktion in Lösung geht. Das verbliebene Natrium (0.2 g) wird entnommen und das THF im Vak. entfernt. Der Rückstand wird in 160 ml abs. entgastem, auf 0°C vorgekühltem DMF gelöst, auf -30°C abgekühlt und auf einmal mit 13.6 g (40 mmol) des Hydrobromids aus $b_2$) versetzt. Das Reaktionsgemisch wird für ca. 30 Min. zäh, um dann wieder dünnflüssiger zu werden. Der Ansatz wird über Nacht bei 0°C unter Feuchtigkeitsausschluß im Kühlschrank aufbewahrt. Das DMF wird im Ölpumpenvak. bei 40°C Badtemperatur abgezogen. Der feste, rötliche, zähe Rückstand wird mit 140 ml Wasser und 140 ml Ether (beide entgast) versetzt, die organische Phase abgetrennt, die Wasserphase noch einmal mit 70 ml Ether extrahiert und die vereinigten Etherphasen mit 160 ml 1N Salzsäure über Nacht unter Argon gerührt. Der weiße Feststoff wird abgesaugt, im HV getrocknet und dann aus 170 ml Isopropanol (entgast) umkristallisiert. Die Kristalle werden abgesaugt, mit Isopropanol nachgewaschen und im HV getrocknet.

Ausbeute: 12.7 g (60 % d. Th.). Bei ca. 200 Torr im Rohr eingeschmolzene Kristalle zeigen einen Schmp. von 238-243°C, beim Aufheizen im geöffneten Schmelzpunktrohr wird Schmp. 224-228°C gefunden.
$[\alpha]_D^{20}$ = +171.6° (c=2.67 in 99 %igem Ethanol).
$^1$H-NMR ($CDCl_3$) : $\delta$ = 10.02 (br,s,2H,$NH_2^+$), 7.30 (m,4H,arom.-H), 6.93 - 7.18 (m,8H,arom.-H), 3.58 (m,2H,$C\underline{H_2}NH$), 3.12 (m,2H,$C\underline{H_2}NH$), 2.88 (m,2H,$C\underline{H}Ar_2$), 2.36 (s,6H,$CH_3$), 2.31 (s,6H,$CH_3$) ppm.

$b_4$) (3R,4R)(+)-1-Acetyl-3,4-bis(di-p-tolylphosphino)-pyrrolidin

3.20 g (6.0 mmol) des Hydrochlorids ($b_3$) und 8 ml (47.0 mmol) Triethylamin werden in 40 ml entgastem Dichlormethan gelöst. Unter Eiskühlung werden 0.57 ml (628 mg, 8.0 mmol) Acetylchlorid innerhalb von 5 Min. zugegeben. Man rührt 2 Std. bei Raumtemperatur, gibt 100 ml 2N Natronlauge zu und rührtweitere 15 Min. Die organische Phase wird mit Wasser, 2N Salzsäure und Wasser gewaschen, getrocknet, das Lösungsmittel bis auf 10 ml im Vak. entfernt und das Produkt durch Zutropfen von 40 ml n-Pentan ausgefällt. Es wird abgesaugt und im HV getrocknet. Ausbeute: 2.94 g (5.47 mmol, 91 % d.Th.), Schmp. 164-167°C,

$[\alpha]_D^{20}$ = +118° (c=0.036 in Toluol).

$b_5$) [(3R,4R)-1-Acetyl-3,4-bis(di-p-tolylphoshino)-pyrrolidin-P,P'](1,5-cyclooctadien)-rhodium-tetrafluoroborat

Zur Lösung von 347 mg (0.85 mmol) Bis(1,5-cyclooctadien)rhodium-tetrafluoroborat (Aldrich) in 3 ml entgastem Dichlormethan gibt man bei Raumtemperatur die Lösung von 460 mg (0.85 mmol) des Acetyl-pyrrolidins aus $b_4$) in 9 ml entgastem Dichlormethan. Man rührt 2 Std. unter Argon, engt im Vak. zum halben Volumen ein und fällt das Produkt durch Zugabe von Ether aus. Es wird unter Argon abgesaugt, in der Mindestmenge Methanol gelöst und durch Zugabe der 50-fachen Menge entgasten Ethers erneut ausgefällt. Man saugt ab und trocknet im HV. Man erhält 620 mg (0.74 mmol, 86 % d.Th.) gelben Feststoff.

$^1$H-NMR ($CD_2Cl_2$): $\delta$ = 7.77 (angenähert t,4H,arom.-H), 7.25-7.50 (m,12H,arom.-H), 5.65 (m,2H,olef.-H), 4.53 (m,2H,olef.-H), 3.75 (m,1H,$NCH_2$), 3.75 (angenähert t,1H,$NCH_2$), 2.70-3,22 (m,4H,2mal C<u>H</u>$PAr_2$ und 2mal $NCH_2$), 2.52 (s,3H,$CH_3$), 2.50 (s,3H,$CH_3$), 2.47 (s,3H,$CH_3$), 2.45 (s,3H,$CH_3$), 2.35-2.55 (m,4H, allyl-H von COD), 2.10-2.22 (m,4H, allyl-H von COD), 1.78 (s,3H,CO-$CH_3$).

c) (3R,4R)(+)-1-Phenylaminocarbonyl-3,4-bis(di-p-tolyl-phosphino)-pyrrolidin (Formel 32)

$c_1$) (3R,4R)(+)-3,4-Bis-(di-p-tolyl-phosphino)-pyrrolidin

Zum entgasten 2-Phasengemisch von 110 ml Diethylether und 27 ml (5.4 mmol) 0.2 N Kalilauge werden 2.02 g (3.80 mmol) des Hydrochlorids aus $b_3$) gegeben und unter Argon gerührt, bis vollständige Lösung eintritt (ca. 10 Min.). Die Etherphase wird abgetrennt, die wäßrige Phase mit 50 ml entgastem Diethylether extrahiert. Die vereinigten Etherphasen werden getrocknet, das Lösungsmittel im Vak. entfernt und der Rückstand im HV getrocknet. Man erhält 1.95 g (3.9 mmol, Ausbeute 100 %) weißen Schaum, Schmp. 63-65°C.

$^1$H-NMR ($CD_3OD$): $\delta$ = 7.28 (t,4H,arom.-H), 7.14 (d,4H,arom.-H), 6.90-7.16 (m,8H,arom.-H), 4.83 (s,1H,NH), 3.23-3.40 (m,1H,$Ar_2$PC<u>H</u>), 2.70-2.88 (m,4H,$NCH_2$), 2.28-2.42 (m,1H,$Ar_2$PC<u>H</u>), 2.30 (s,12H,$CH_3$) ppm.

Die Verbindung wurde ohne weitere Reinigung umgesetzt.

$c_2$) (3R,4R)(+)-1-Phenylaminocarbonyl-3,4-bis(di-p-tolyl-phosphino)-pyrrolidin

Zur Argon-durchperlten Lösung von 1.87 g (4.0 mmol) des Produkts aus $c_1$) in 30 ml Dichlormethan tropft man unter Eiskühlung 500 mg (4.2 mmol) Phenylisocyanat und rührt dann 30 Min. bei Raumtemperatur. Man tropft 10 ml Wasser zu, rührt 15 Min., trennt die wäßrige Phase ab und wäscht die organische Phase nach Verdünnen mit 60 ml entgastem Dichlormethan mit zweimal je 20 ml 2N Salzsäure, dann mit 20 ml Wasser. Man trocknet die organische Phase, entfernt das Lösungsmittel im Vak. und trocknet den Rückstand im HV. Man erhält 2.08 g (3.55 mmol, 89 % d.Th.) Rohprodukt. Dieses wird in 5 ml Dichlormethan gelöst und 40 ml entgastes n-Hexan zugetropft, wobei eine bleibende Trübung eintritt. Die Suspension wird 12 Std. auf 0°C gekühlt, der Feststoff abgesaugt, mit n-Hexan gewaschen und im HV getrocknet. Man erhält 1.92 g (3.12 mmol, 83 % d.Th.) farblosen Feststoff, $[\alpha]_D^{20}$ = 93.2° (c=2.04, Benzol).

Beim Erwärmen erfolgt Zersetzung unter Gasentwicklung bei 110-120°C. DC: Cyclohexan/Ethylacetat 3 : 1, $R_f$: 0.40.

$^1$H-NMR (DMSO-$d_6$): $\delta$ = 8.14 (s,1H,NH), 6.85-7.48 (m,21H,arom.-H), 3.84 (m,2H,$NCH_2$), 3.38 (t,2H,$NCH_2$), 2.85 (t,2H,C<u>H</u>$PAr_2$), 2.30 (s,6H, 2x$CH_3$), 2.28 (s,6H, 2x$CH_3$) ppm.

$c_3$) [(3R,4R)(+)-1-Phenylaminocarbonyl-3,4-bis(di-p-tolyl-phosphino)-pyrrolidin-P,P'](1,5-cyclooctadien)rhodium-tetrafluoroborat

Zur Lösung von 630 mg (1.55 mmol) Bis(1,5-cyclooctadien)rhodium-tatrafluoroborat in 6 ml entgastem Dichlormethan gibt man unter Argon bei Raumtemperatur die Lösung von 935 mg (1.52 mmol) des Produkts aus $c_2$) in 23 ml Dichlormethan. Man rührt 2 Std. DC ($CH_2Cl_2$/$CH_3OH$) zeigt quantitative Umsetzung des Phosphins ($R_f$=0.81) zum Rhodium-Komplex ($R_f$=0.43) an. Das Lösungsmittel wird im Vak. entfernt, der Rückstand in 6 ml Methanol aufgenommen und der Suspension 150 ml Ether zugesetzt. Man rührt 15 Min. unter Argon, saugt den Feststoff ab, wäscht ihn mit Ether und trocknet ihn im HV. Man erhält 1.35 g (1.48 mmol, 97 % d.Th.) orangegelbes Pulver.

$^1$H-NMR ($CD_2Cl_2$): sehr komplexes, reproduzierbares Spektrum.
$^{31}$P-NMR ($CD_2Cl_2$, gegen 85 %iges $H_3PO_4$ als externer Standard).
$\delta$ = 32.8 ppm (d, $J_{Rh,P}$=150.3 Hz, Halbwertsbreite ca. 30 Hz).

d) (3R,4R)(+)-1-Phenylaminocarbonyl-3,4-bis-(diphenylphosphino)-pyrrolidin (Formel 31)
$d_1$) (3R,4R)(+)-3,4-Bis-(diphenylphosphino)-pyrrolidiniumchlorid
wird in Analogie zu $b_3$) hergestellt. Ausbeute 74 % d.Th., Schmp. 207-209°C,

$[\alpha]_D^{25}$ = +163° (c=3.0 in 99 %igem Ethanol).
$^1$H-NMR ($CDCl_3$): $\delta$ = 9-11 (s,sehr breit,2H,$NH_2^+$), 7.1-7.6 (m,20H,arom.-H), 3.62 (m,2H,$NCH_2$), 3.16 (m,2H,$NCH_2$), 2.95 (m,2H,C<u>H</u>$PPh_2$).

$d_2$) (3R,4R)(+)-3,4-Bis-(diphenylphosphino)-pyrrolidin
wird aus $d_1$) in Analogie zu $c_1$) hergestellt. Das Rohprodukt hat Schmp. 120-122°C.

$^1$H-NMR ($CD_3OD$): $\delta$ = 7.1-7.5 (m,20H,arom.-H), 4.82 (s,1H,NH), 3.30-3.45 (m,2H,C<u>H</u>$PPh_2$), 2.75-2.93 (m,4H,$CH_2$) ppm.

$d_3$) (3R,4R)(+)-1-Phenylaminocarbonyl-3,4-bis(diphenylphosphino)pyrrolidin
wird aus dem Rohprodukt $d_2$) in Analogie zu $c_2$) hergestellt. Ausbeute: 100 %, Schmp. 165-167°C, DC (Cyclohexan/Ethylacetat 3 : 2) $R_f$=0.48.

$^1$H-NMR (DMSO-$d_6$): $\delta$ = 8.20 (s,1H,NH), 7.05-7.50 (m,24H,arom.-H), 6.90 (m,1H,arom.-H), 3.90 (m,2H,$NCH_2$), 3.43 (t,2H,$NCH_2$, 2.92 (t,2H,C<u>H</u>$PPh_2$) ppm.

$d_4$) [(3R,4R)(+)-1-Phenylaminocarbonyl-3,4-bis(diphenylphosphino)-pyrrolidin-P,P']-(1,5-cyclooctadien)-rhodium-tetrafluoroborat
wird aus dem Produkt $d_3$) in Analogie zu $c_3$) hergestellt. Gelborangenes Pulver, Ausbeute: 91 % d.Th.

MS (FAB, 3-NBA): m/e = 769 (Molekülpeak des Kations).
$^1$H-NMR ($CD_2Cl_2$): $\delta$ = 7.98 (m,4H,arom-H), 7.52-7.80 (m,12H,arom.-H), 7.45 (m,4H,arom.-H), 7.31 (dd,2H,arom.-H), 7.15 (t,2H,arom.-H), 6.91 (m,angenähert t, 1H, arom.-H), 6.64 (s,1H,NH), 5.20 (s,breit, 2H,olef.-H), 4.56 (qua,2H,olef.-H), 3.75 (m,2H,$NCH_2$), 3.14 (m,2H,$NCH_2$), 2.99 (m,2H,C<u>H</u>$PPh_2$), 2.30-2.65 (m,4H,allyl-H von COD), 2.05-2.30 (m,4H, allyl-H von COD) ppm.
$^{31}$P-NMR ($CD_2Cl_2$ gegen 85 %ige $H_3PO_4$ als externer Standard): $\delta$ = 34.1 ppm (d, $J_{Rh,P}$= 149.0 Hz,Halbwertsbreite ca.30 Hz).

Beispiel 6

Herstellung von im Prinzip literaturbekannten Ruthenium-Komplexen chiraler Diphosphine

e) [((S)-BINAP-P,P')($\mu$-iodo)($\eta$-p-cymol)]-ruthenium(II)-iodid (allgemeine Formel 16 mit $Y^1 = Y^{1(2)}$ = I, $R^7$ = $CH_3$, $R^8$ = i-Pr, Diphosphin-Ligand = (S)(-)-BINAP der Formel 33):
Der Komplex wird gemäß den prinzipiellen Angaben (dort keine experimentellen Daten) von K. Mashima et al., J. Chem. Soc., 1208 (1989) hergestellt.
$e_1$) Di-$\mu$-iodo-diiodo-bis[(p-cymol)-ruthenium(II)] alternativer Name: [(p-Cymol)-Ruthenium(II)-iodid]-Dimer
Eine Suspension von 5.23 g (20.0 mmol) Ruthenium(III)-chlorid-Trihydrat (Riedel-de Haen) und 10.90 g (80.0 mmol) (R)(-)-5-Isopropyl-2-methyl-1,3-cyclohexadien (Merck-Schuchardt, alternative Namen: (R)(-)-$\alpha$-Phellandren; p-Mentha 1,5-dien) in 100 ml 90 %igem Ethanol wird unter Argon 5 Std. auf 50°C erwärmt, wobei die schwarze Suspension in eine orangefarbene Lösung übergeht. Die Lösung wird innerhalb 30 Min. in die Lösung von 16.6 g (100.0 mmol) Kaliumiodid in 70 ml 50 %igem Ethanol getropft. Der ausgefallene luftstabile Feststoff wird nach einer Std. mit einer Glasfilterfritte abgesaugt, mit 50 %igem Ethanol gewaschen und kurz im HV getrocknet. Man erhält 6.7 g (6.85 mmol, 68 % d.Th.) purpurfarbenes Pulver, Schmp. 226-229°C (unter Zersetzung).

$^1$H-NMR (DMSO-$d_6$): $\delta$ = 5.87 (AB-System,8H,arom.-H), 3.15 (sept.,2H,C<u>H</u>$(CH_3)_2$), 2.39 (s,6H,$CH_3$),1.21 (d,12H,CH(C<u>H</u>$_3$)$_2$) ppm.
MS (FAB): Büschel von Isotopensignalen für $(M - I)^+ = C_{20}H_{28}I_3Ru_2$: m/e = 858 (Int.4 %), 857 (20 %), 856 (15 %), 855 (73 %), 854 (55 %), 853 (100 %), 852 (99 %), 851 (78%), 850 (77 %), 849 (56 %), 848 (24 %), 847 (30 %), 846 (12 %), 845 (9 %), 844 (7 %). Die Intensitätsverteilung der Isotopenpeaks war identisch mit einer Computer-Simulation für die obige Summenformel.

$e_2$) [((S)(-)-BINAP-P,P')(μ-iodo)(n-p-cymol)]-ruthenium(II)iodid

Zu dem Gemisch aus 157 mg (0.16 mmol) des Produkts aus $e_1$) und 200 mg (0.32 mmol) (S)(-)-BINAP (Aldrich) wird unter Argon 20 ml eines entgasten Ethanol/Dichlormethan (4 : 1)-Gemischs gegeben. Anschließend setzt man tropfenweise (ca. 3 ml) Dichlormethan zu, bis vollständige Lösung des Feststoffs eintritt. Man rührt 1 Std. bei 45°C. DC ($CH_2Cl_2$/EtOAc 1 : 1) zeigt vollständige Umsetzung von BINAP ($R_f$=0.79) und dem Komplex aus $e_1$) ($R_f$=0.58) zum Produkt ($R_f$=0.35) an. Das Lösungsmittel wird im Vak. entfernt und der zurückbleibende Feststoff mit 30 ml Ethanol/Dichlormethan (4 : 1) extrahiert. Ungelöster Feststoff wird abfiltriert und das Filtrat im Vak. aufkonzentriert. n-Hexan wird bis zur deutlichen Trübung zugetropft. Man läßt 12 Std. bei 0°C kristallisieren, saugt ab, wäscht mit n-Hexan und trocknet kurz im HV. Man erhält 270 mg (0.24 mmol, 75 % d.Th.) braunes Pulver, Schmp. 225-227°C (unter Zersetzung).

IR (KBr): 3050 (schwach), 1433 (stark), 742 (stark), 697 (stark) $cm^{-1}$.
$^{31}$P-NMR ($CDCl_3$, gegen 85 %iges $H_3PO_4$ als externer Standard): $\delta$ = 41.6 ppm (P, d, $J_{P,P'}$ = 60.0 Hz), 24.7 ppm (P', d, $J_{P,P'}$ = 60.0 Hz).

f) [(S)-BINAP]-Ruthenium(II)-dialkanoat-Komplexe der allgemeinen Formel 19 werden entsprechend den Vorschriften von T. Ohta, H. Takaya, R. Noyori, Inorg. Chem. 27, 566 (1988) und von M.T. Ashby, J. Halpern J. Am. Chem. Soc. 113, 589 (1991) hergestellt.
g) Ruthenium-Komplexe der allgemeinen Formel 18 werden entsprechend den Vorschriften in H. Kawano et al., J. Chem. Soc. Perkin Trans. I, 1571 (1989) hergestellt.

**Patentansprüche**

1. Verfahren zur Herstellung von 2(R)-Benzylbernsteinsäure-Derivaten der Formel (1)

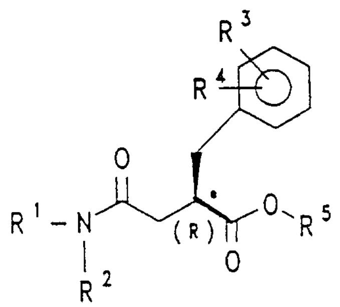

(1)

wobei

$R^1$ und $R^2$

a) gleich oder verschieden sind und geradkettiges oder verzweigtes Alkyl mit 1 bis 7 Kohlenstoffatomen oder Cycloalkyl mit 5 bis 7 Kohlenstoffatomen bedeuten, wobei die genannten Gruppen substituiert sein können mit bis zu drei gleichen oder verschiedenen Resten aus der Reihe

- ($C_1$-$C_7$)-Alkyl
- Hydroxy (geschützt oder ungeschützt)
- ($C_1$-$C_7$)-Alkoxy
- Amino (geschützt oder ungeschützt)
- ($C_1$-$C_7$)-Alkylamino (geschützt oder ungeschützt)
- Di-($C_1$-$C_7$)-Alkylamino oder

$R^1$ und $R^2$ gemeinsam können unter Ausbildung eines 5- bis 7-gliedrigen Ringes, von dem 0,1 oder 2 Ringglieder gleich oder verschieden aus Sauerstoff- oder Stickstoffatomen bestehen, miteinander verbunden sein, der gegebenenfalls mit den obengenannten Resten substituiert sein kann,
b) $R^1$ und $R^2$ können auch die Bedeutung von Phenylgruppen haben, die gegebenenfalls substituiert sein können mit Gruppen, die der unten angegebenen Bedeutung von $R^3$ und $R^4$ entsprechen,

$R^3$ und $R^4$ gleich oder verschieden sind und Wasserstoff, Trifluormethyl, Halogen oder die bei $R^1$ und $R^2$ unter a) genannten Substituenten bedeuten und

$R^5$ ein Wasserstoffatom bedeutet,

wobei man

a) ein Phenylitaconsäure-Derivat der Formel (4)

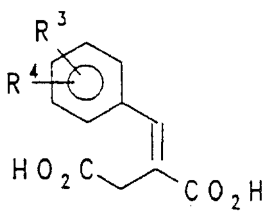

(4)

in das Anhydrid der Formel (7) überführt, wobei $R^3$ und $R^4$ die obengenannte Bedeutung haben

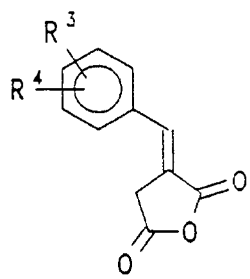

(7)

b) dieses Anhydrid der Formel (7) in einer regioselektiven Reaktion mit einem Amin der Formel $R^1R^2NH$ zum Monoamid der Formel (8) umsetzt, wobei $R^1$, $R^2$, $R^3$ und $R^4$ die obengenannten Bedeutungen haben, und

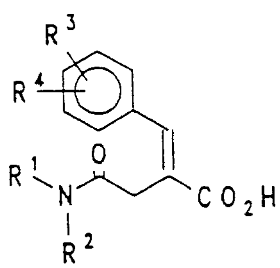

(8)

c) dieses Monoamid der Formel (8) in Gegenwart eines chiralen Rhodium(I)- oder Ruthenium(II)-Diphosphin-Katalysators, dessen Chiralität nicht ausschließlich auf der Anwesenheit chiraler Phosphor-Atome beruht, asymmetrisch zum 2(R)-Benzylbernsteinsäure-Derivat (1) hydriert.

**2.** Verfahren gemäß Anspruch 1, dadurch gekennzeichnet, daß man eine Verbindung der Formel (1) herstellt, bei der die Symbole folgende Bedeutung haben:

$-NR^1R^2$ gleich Pyrrolidinyl

Pyrazolidinyl
Imidazolidinyl
Piperidinyl
Piperazinyl
Tetrahydropyrimidinyl
3-Hydroxypiperidinyl
4-Hydroxypiperidinyl
3-Aminopiperidinyl
4-Aminopiperidinyl
Morpholinyl
1,4-Diazacycloheptyl,

wobei die Hydroxy- und Aminofunktionen jeweils geschützt oder ungeschützt sein können,

$R^3$, $R^4$, $R^5$ gleich Wasserstoff.

**Claims**

1. A process for the preparation of 2(R)-benzylsuccinic acid derivatives of formula (1):

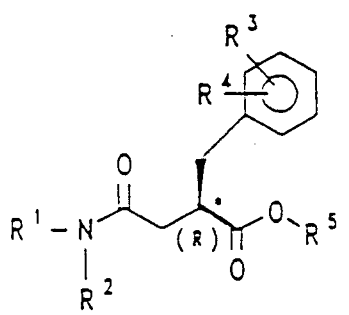

(1)

in which

$R^1$ and $R^2$

a) are identical or different and are linear or branched alkyl having 1 to 7 carbon atoms or cycloalkyl having 5 to 7 carbon atoms, it being possible for said groups to be substituted by up to three identical or different radicals selected from the group comprising

- $(C_1-C_7)$-alkyl
- hydroxyl (protected or unprotected)
- $(C_1-C_7)$-alkoxy
- amino (protected or unprotected)
- $(C_1-C_7)$ -alkylamino (protected or unprotected)
- di-$(C_1-C_7)$-alkylamino, or

$R^1$ and $R^2$ can be bonded together to form a 5- to 7-membered ring of which 0, 1 or 2 ring members are identical or different and are oxygen or nitrogen atoms, it being possible for the ring to be unsubstituted or substituted by the aforementioned radicals, or

b) $R^1$ and $R^2$ can also be phenyl groups which can be unsubstituted or substituted by groups having the meanings given below for $R^3$ and $R^4$,

$R^3$ and $R^4$ are identical or different and are hydro gen, trifluoromethyl, halogen or the substituents mentioned for $R^1$ and $R^2$ under a), and

$R^5$ is a hydrogen atom,

which comprises

a) converting a phenylitaconic acid derivative of formula (4):

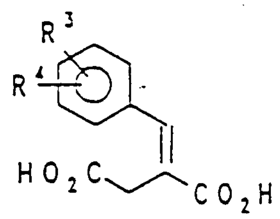

(4)

to the anhydride of formula (7):

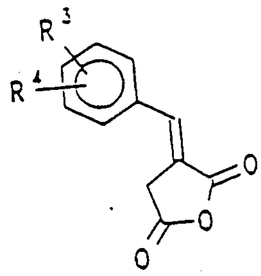

(7)

$R^3$ and $R^4$ being as defined above,

b) converting this anhydride of formula (7) to the monoamide of formula (8) :

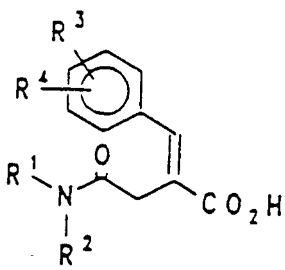

(8)

in a regioselective reaction using an amine of the formula $R^1R^2NH$=, $R^1$, $R^2$, $R^3$ and $R^4$ being as defined above, and

c) asymmetrically hydrogenating this monoamide of formula (8) in the presence of a chiral rhodium(I)- or ruthenium(II)-diphosphine catalyst, the chirality of which is not exclusively based on the presence of chiral phosphorus atoms, to give the 2(R)-benzylsuccinic acid derivative (1).

**2.** The process as claimed in claim 1, wherein a compound of formula (1) is prepared in which the symbols have the following meanings:

$-NR^1R^2$ is pyrrolidinyl
pyrazolidinyl
imidazolidinyl
piperidinyl
piperazinyl
tetrahydropyrimidinyl
3-hydroxypiperidinyl

4-hydroxypiperidinyl
3-aminopiperidinyl
4-aminopiperidinyl
morpholinyl
1,4-diazacycloheptyl,

it being possible for all the hydroxyl and amino groups to be protected or unprotected, and

$R^3$, $R^4$ and $R^5$ are hydrogen.

## Revendications

1. Procédé de préparation de dérivés d'acide 2(R)-benzylsuccinique de formule (1)

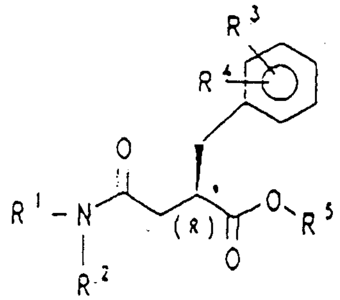

(1)

dans laquelle

$R^1$ et $R^2$

a) sont identiques ou différents et représentent un groupe alkyle à chaîne linéaire ou ramifiée avec 1 à 7 atomes de carbone ou cycloalkyle avec 5 à 7 atomes de carbone, lesdits groupes pouvant être substitués avec jusqu'à trois radicaux identiques ou différents choisis parmi les groupes

- alkyle en $C_1$-$C_7$
- hydroxy (protégé ou non protégé)
- alcoxy en $C_1$-$C_7$
- amino (protégé ou non protégé)
- alkylamino en $C_1$-$C_7$(protégé ou non protégé)
- dialkylamino en $C_1$-$C_7$ ou

$R^1$ et $R^2$ peuvent être liés l'un avec l'autre pour former un noyau de 5 jusqu'à 7 membres, dans lequel 0, 1 ou 2 membres du noyau identiques ou différents sont constitués d'atomes d'oxygène ou d'azote, qui peut éventuellement être substitué par les radicaux ci-dessus mentionnés,

b) $R^1$ et $R^2$ peuvent aussi représenter des groupes phényle qui peuvent être éventuellement substitués avec des groupes qui correspondent à la signification donnée ci-dessous pour $R^3$ et $R^4$,

$R^3$ et $R^4$ sont identiques ou différents et représentent un atome d'hydrogène, un groupe trifluorométhyle, un atome d'halogène ou les substituants cités en a) pour $R^1$ et $R^2$ et

$R^5$ représente un atome d'hydrogène,

procédé dans lequel

a) on transforme un dérivé de l'acide phénylitaconique de formule (4)

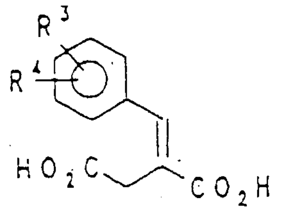

( 4 )

dans l'anhydride de formule (7), formules dans lesquelles $R^3$ et $R^4$ ont les signification ci-dessus indiquées

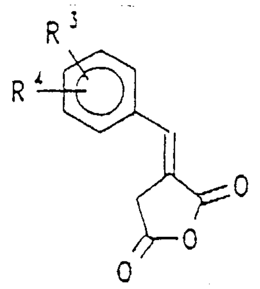

( 7 )

b) on fait réagir cet anhydride de formule (7) dans une réaction régiosélective avec une amine de formule $R^1R^2NH$ pour donner un monoamide de formule (8), dans laquelle $R^1$, $R^2$, $R^3$ et $R^4$ ont la signification ci-dessus mentionnée, et

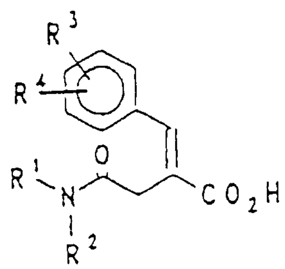

( 8 )

c) on effectue l'hydrogénation de ce monoamide de formule (8) en présence d'un catalyseur chiral de diphosphine de rhodium(I) ou de ruthénium(II) dont la chiralité ne repose par exclusivement sur la présence d'atomes de phosphore chiraux, de manière asymétrique en dérivé de l'acide 2(R)-benzylsuccinique (1).

**2.** Procédé selon la revendication 1, caractérisé en ce qu'on prépare un composé de formule (1), dans lequel les symboles ont les significations suivantes : le groupement $-NR^1R^2$ est le groupe

- pyrrolidinyle
- pyrazolidinyle
- imidazolidinyle
- pipéridinyle
- pipérazinyle
- tétrahydropyrimidinyle
- 3-hydroxypipéridinyle
- 4-hydroxypipéridinyle
- 3-aminopipéridinyle
- 4-aminopipéridinyle

- morpholinyle
- 1,4-diazacycloheptyle,

dans lequel les fonctions hydroxy et amino peuvent être respectivement protégées ou non protégées,

$R^3$, $R^4$, $R^5$ représentent un atome d'hydrogène.